# EUROPEAN PATENT SPECIFICATION

(11) **EP 3 709 899 B1**
(45) Date of publication and mention of the grant of the patent: **22.04.2026**
(21) Application number: 18878175.1
(22) Date of filing: 16.11.2018
(51) Int. Cl.: A61B 17/12, A61B 17/04, A61B 17/00

(54) **SYSTEM FOR LEFT ATRIAL APPENDAGE CLOSURE**
SYSTEM ZUM VERSCHLUSS DES LINKEN HERZOHRS
SYSTÈME DE FERMETURE D'APPENDICE AURICULAIRE GAUCHE

(30) Priority: 17.11.2017 US 201762588225 P; 17.11.2017 US 201762588243 P
(43) Date of publication of application: 23.09.2020
(73) Proprietor: Surecor, Inc., Fremont, California 94555 (US)
(72) Inventor: TUN, Zaya, Livermore California 94551 (US); QUINTOS, Robert, Newark California 94560 (US); SABRAMANIAM, Raj, Fremont California 94555 (US)
(74) Representative: Mathys & Squire
(86) International application number: PCT/US2018/061612
(87) International publication number: WO 2019/099896

(56) References cited:
- WO-A1-2012/091809
- WO-A1-2017/066197
- CN-A- 107 233 117
- US-A- 6 096 525
- US-A1- 2004 127 935
- US-A1- 2004 127 935
- US-A1- 2009 226 600
- US-A1- 2010 324 585
- US-A1- 2010 324 585
- US-A1- 2014 277 074
- US-A1- 2015 313 604
- US-A1- 2016 100 844
- US-A1- 2017 095 256
- US-B2- 7 597 704
- US-B2- 9 186 152

## Description

### CLAIM OF PRIORITY

The present application is a non-provisional of, and claims the benefit of US Provisional Patent Application Nos. 62/588,225 filed November 17, 2017 and 62/588,243 also filed November 17, 2017.

### BACKGROUND

Millions of individuals world-wide have atrial fibrillation. Patients with this irregular heart beat are at risk from stroke because the irregular heart beat can cause blood to be stagnant in certain parts of the heart creating thrombus, which can then embolize to the brain, lungs or other parts of the body resulting in a stroke. More often the thrombus forms in the left atrial appendage (LAA) which is a pouch-like extension of the heart muscle with unknown function.

Prophylactic treatment to reduce the risk of stroke often includes the use of anticoagulant therapies such as Coumadin or other Novel Oral Anti-Coagulants (NOAC) blood thinners.

Surgical exclusion of the LAA is also a treatment option that is very effective in reducing the risk of stroke in patients with atrial fibrillation (AF). More recent treatments include the use of minimally invasive left atrial appendage closure devices to prevent blood from clotting in the LAA and embolization of those clots. These devices are often delivered to the heart with a catheter and the device expands into the LAA.

US 2010/324585 describes devices, methods and systems for occluding an opening within the tissue of a body, such as a left atrial appendage. A system includes a handle, a catheter coupled with the handle and a medical device disposed within a lumen of the catheter. The medical device includes an anchor system having a plurality of anchor segments coupled with an anchor hub. The medical device further includes an occluder system having a plurality of occluder segments coupled with an occluder hub. A first actuator is configured to displace the occluder hub independent of, and relative to, the anchor hub to deploy the occluder system from a refracted state to an expanded state while the anchor system remains in a retracted state.

US 2004/127935 describes apparatus for permanent placement across an ostium of a left atrial appendage in a patient, which includes a filtering membrane configured to extend across the ostium of the left atrial appendage. The filtering membrane has a permeable structure which allows blood to flow through but substantially inhibits thrombus from passing therethrough. The apparatus also includes a support structure comprising a plurality of fingers which are radially outwardly expandable with respect to a longitudinal axis to permanently engage the interior wall of the left atrial appendage, wherein the filtering membrane is attached to the support structure extending across the ostium of the left atrial appendage.

WO 2012/091809 describes an occlusive device for left atrial appendage occlusion that has a membrane component configured to inhibit passage of blood and an expandable frame formed from a plurality of wires having a cupped occlusive component at least partially covered with the membrane component, one or more anchors with looped ends and a hub component. The occlusive device can be delivered percutaneously. The occlusive device is useful in the occlusion of the left atrial appendage.

WO 2017/066197 describes devices and methods for occluding the left atrial appendage (LAA) to exclude the LAA from blood flow to prevent blood from clotting within the LAA and subsequently embolizing, particularly in patients with atrial fibrillation. A foam implant is delivered via transcatheter delivery into the LAA and anchored using an internal locking system of the implant. The locking system includes deployable anchors that can be deployed after deployment of the foam implant from the delivery catheter and expansion of the foam within the LAA. The locking system can be reversible to allow retraction of the anchors and repositioning or retrieval of the implant.

US 2016/100844 describes a left atrial appendage occluder which comprises an elastic closure disc, and a supporting structure connecting with the closure disc and being located on one side of the closure disc, with the supporting structure comprising a central end connecting with the closure disc and a plurality of interconnected and bent struts, wherein at least one anchoring thorn is set near the end of at least one strut with the anchoring thorn toward the closure disc.

CN 107 233 117 describes a left atrial plugging device, which comprises a plugging disc, a fixing disc and a connecting device, wherein the plugging disc is used for plugging the left atrium; the fixing disc is used for fixing the left atrium plugging device on the left atrium; barbs are arranged on the fixing disc; the connecting device connects the plugging disc and the fixing disc; the connecting device comprises a dismountable adjusting structure.

The present invention is set out in the appended claims.

### BRIEF DESCRIPTION OF THE DRAWINGS

In the drawings, which are not necessarily drawn to scale, like numerals may describe similar components in different views. Like numerals having different letter suffixes may represent different instances of similar components. The drawings illustrate generally, by way of example, but not by way of limitation, various embodiments discussed in the present document.
Fig. 1 illustrates the basic anatomy of a heart.
Figs. 2A-2C illustrate various exemplary shapes of a left atrial appendage.
Fig. 3A illustrates a schematic diagram of a basic left atrial appendage closure device.
Fig. 3B illustrates implantation of the device from Fig. 3A into the heart.
Fig. 4 illustrates an exemplary sealing element.
Figs. 5A-5C illustrate exemplary positions of the sealing element relevant to the ostium of the left atrial appendage.
Figs. 6A-6B show the sealing element in the collapsed and expanded configurations.
Fig. 7 illustrates an example of an anchor element.
Fig. 8 illustrates an example of an anchor element.
Fig. 9 illustrates an example of a left atrial appendage closure device.
Figs. 10A-10C illustrate another example of a left atrial appendage closure device.
Fig. 11 illustrates a left atrial appendage closure device and a delivery system.
Fig. 12 illustrates another example of an anchor element.
Fig. 13 illustrates still another example of an anchor element.
Fig. 14 illustrates another example of an anchor element.
Figs. 15A-15D illustrate another exemplary closure device.
Figs. 16A-16F illustrate an exemplary method of sealing a left atrial appendage.
Figs. 17A-17B illustrate another example of a left atrial appendage closure device and a delivery system.
Fig. 18A illustrates an example of a left atrial appendage closure device in the expanded configuration.
Fig. 18B illustrates the device of Fig. 18A in the collapsed configuration.
Fig. 19 illustrates the device of Fig. 18B coupled to a delivery system.
Fig. 20 illustrates a portion of a delivery system.
Figs. 21 illustrates another portion of a delivery system.
Fig. 22 illustrates another example of a left atrial appendage closure device.
Fig. 23 illustrates the device of Fig. 22 in a linear configuration.
Fig. 24 illustrates a flat pattern of a sealing portion of the device.
Fig. 25 illustrates a flat pattern of a sealing portion of the device.
Fig. 26 illustrates a partial flat pattern of an anchoring portion of the device.
Figs. 27A and 27B illustrate partial flat patterns of an anchoring portion of the device.
Fig. 28 illustrates a flat pattern of a sealing portion of the device.
Fig. 29 illustrates flat pattern of an anchoring portion of the device.
Fig. 30 illustrates another example of a closure device.
Figs. 31A-31D illustrate another example of a closure device.
Figs. 32A-32D illustrate a closure device and delivery catheter.
Figs. 33A-33B illustrate deployment of a closure device.
Fig. 34 shows another example of a closure device.
Figs. 35A-35D show another example of a closure device implanted in a LAA.

### DETAILED DESCRIPTION

The present application generally relates to medical devices, systems and methods, and more particularly relates to devices, systems, and methods for left atrial appendage closure. Specific embodiments of the disclosed device, delivery system, and method will now be described with reference to the drawings. Nothing in this detailed description is intended to imply that any particular component, feature, or step is essential to the invention.

Because thrombus often forms in the LAA in patients with atrial fibrillation, it would be desirable to provide devices, systems, and methods for preventing the thrombus from forming in the LAA or embolizing from the LAA.

The use of anticoagulants is challenging since many patients cannot tolerate long term oral anticoagulants and the may experience micro bleeding, possibly in their gastrointestinal system. Furthermore, some anticoagulants (e.g. Coumadin) require routine blood testing to ensure that the proper dosage is being administered and this can present patient compliance issues as well as represents an inconvenience.

Surgical exclusion of the LAA is invasive and requires a lengthy hospital stay. More recent treatments include the use of minimally invasive left atrial appendage closure devices to prevent blood from clotting in the LAA and embolization of those clots. These devices are often delivered to the heart with a catheter and the device expands into the LAA. Sizing and anchoring of these devices can be challenging, and the devices may not properly seal the ostium of the LAA or the devices may migrate out of position. Delivery can also be challenging requiring the proper approach angle. Improperly delivered devices can also cause cardiac tamponade. Imaging with transesophageal echocardiography may be used to help with sizing and delivery but this requires multiple views to properly size the LAA and adds cost and time to the procedure. In a small percent (about 3%) of patients, the device can come loose and embolize. Additionally, implants do not always endothelialize quickly and this may require post-implant anti-thrombotics to prevent clotting which was previously discussed as having its own challenges. It would therefore be advantageous to provide improved LAA closure devices, delivery systems and methods that address at least some of these challenges.

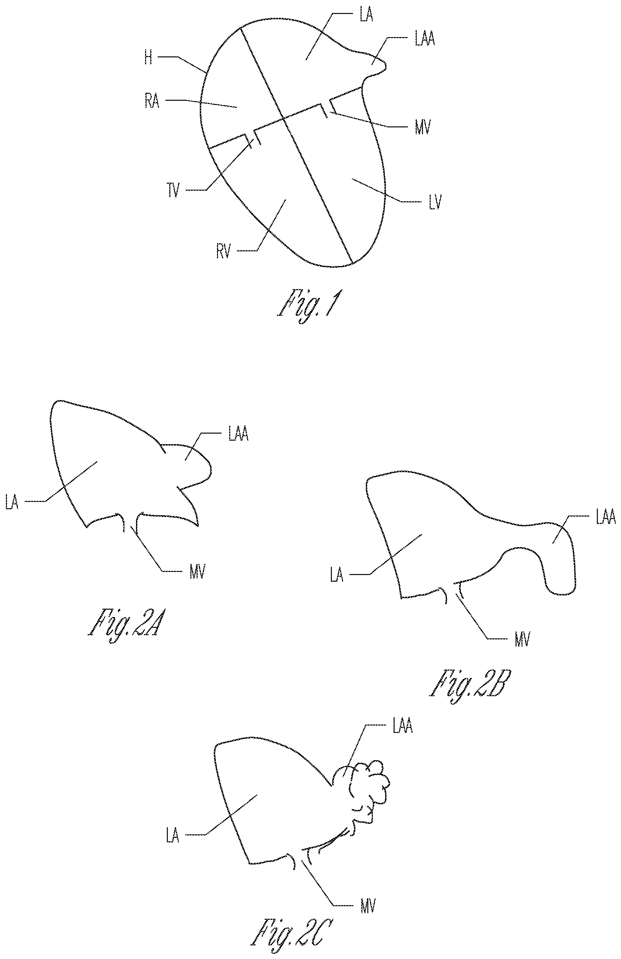

Fig. 1 illustrates the basic anatomy of a human heart H. The heart H includes four chambers including the right atrium RA, right ventricle RV, left atrium LA, and left ventricle LV. Veins and arteries coupled to the heart have been excluded from Fig. 1 for convenience. The tricuspid valve TV prevents regurgitation between the right ventricle and the right atrium, and the mitral valve MV prevent regurgitation between the left ventricle and the left atrium. An area of the left atrium often protrudes outward from the heart forming the left atrial appendage LAA, which is a cavity where blood may pool and potentially form thrombus, especially in patients with atrial fibrillation.

The left atrial appendage LAA may take many forms but roughly can be divided into three general shapes. Figs. 2A-2C illustrate these general shapes.

Fig. 2A illustrates the left atrium LA and mitral valve MV with the left atrial appendage LAA shaped like a windsock. The windsock is generally a tapered cylinder that tapers outward and away from the left atrium. Fig. 2B illustrates the left atrium LA and mitral valve MV with the left atrial appendage shaped like a "chicken wing." The appendage has a first elongate narrow section extending outward from the left atrium and then has a sharp bend with another elongate narrow section extending transversely from the first section. Fig. 2C illustrates another common form of left atrial appendage LAA. Here the left atrium LA and mitral valve MV include a left atrial appendage LAA that is "cauliflower" shaped. The appendage has a plurality of discrete cavities that merge together to form the overall appendage.

Because the left atrial appendage can be many different shapes and sizes, it would be desirable to provide a left atrial appendage closure device that be easily and accurately delivered and deployed into the appendage, and that seals the appendage to prevent blood clots that form from embolizing.

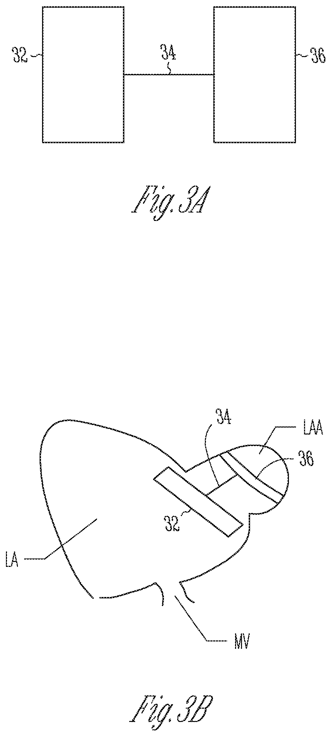

Fig. 3A illustrates a schematic diagram of a basic left atrial appendage closure device. The device includes a sealing element 32 and an anchor element 36. A coupling element 34 joins the sealing element 32 with the anchor 36. The sealing element 32 seals the ostium of the left atrial appendage and prevents thrombus from embolizing out of the appendage. It may be positioned just outside of the ostium, or it may be positioned at the ostium, or it may be positioned just inside of the ostium. The sealing element may be a self-expanding fabric covered frame, such as a self-expanding Nitinol frame covered with PET (polyethylene terephthalate), ePTFE (expanded polytetrafluorinated ethylene), or any other material. The wire frame may have any number of patterns including a woven mesh. The anchoring element 36 secures the device to tissue inside of the left atrial appendage and ensures that the seal is maintained between the sealing element and tissue at or around the ostium, and also prevents the device itself from embolization. The anchoring element 36 may also seal at any other position including just outside the ostium to the appendage or just inside the ostium. The coupling element ensures that the sealing element is coupled to the anchor element. These features may be applied to any of the examples disclosed herein. Fig. 3B shows the sealing device disposed in the left atrial appendage.

### Sealing Element

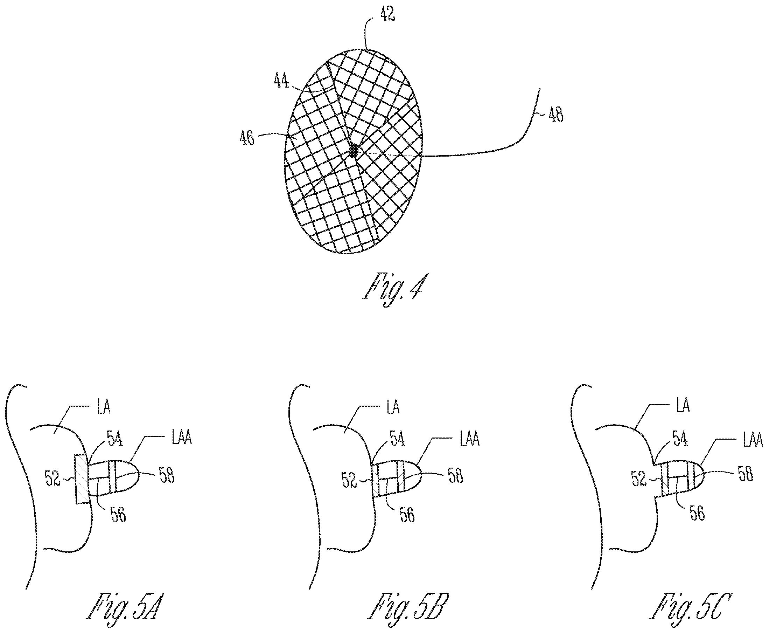

Fig. 4 illustrates an exemplary sealing element 42. The sealing element in this embodiment is a flat planar disc. One or more ribs 44 are disposed within the disc and a covering 46 is disposed over the disc. A coupling element 48 is attached to the sealing element and allows the sealing element to be coupled to the anchoring element (not shown) which may be any anchoring element disclosed herein. The ribs may extend radially outward from the center of the disc in a spoke-like fashion, or other patterns may be used. The ribs are preferably formed from a superelastic or shape memory material such as Nitinol to allow them to be compressed into a collapsed configuration for delivery from a catheter, and then to self-expand into an expanded configuration during deployment. This or any of the embodiments of sealing elements may be formed by a plurality of filaments woven together to form a wire frame. The covering may be a fabric such as Dacron polyester, polyethylene terephthalate (PET), or other materials such as ePTFE or other materials known in the art.

The sealing element 42 is sized and shaped to create a seal that prevents thrombus from embolizing from the appendage. The sealing element may be positioned in various locations around the ostium as seen in Figs. 5A-5B.

Fig. 5A shows the sealing element 52 disposed flush and just outside the ostium 54 of the left atrial appendage LAA in the left atrium LA. A coupling element 56 joins the sealing element 52 with the anchor element 58.

In Fig. 5B the sealing element 52 is disposed at the ostium 54 of the left atrial appendage LAA in the left atrium LA. A coupling element 56 joins the sealing element 52 with the anchor element 58.

In Fig. 5C the sealing element 52 is disposed just inside the ostium 54 of the left atrial appendage LAA in the left atrium LA. A coupling element 56 joins the sealing element 52 with the anchor element 58.

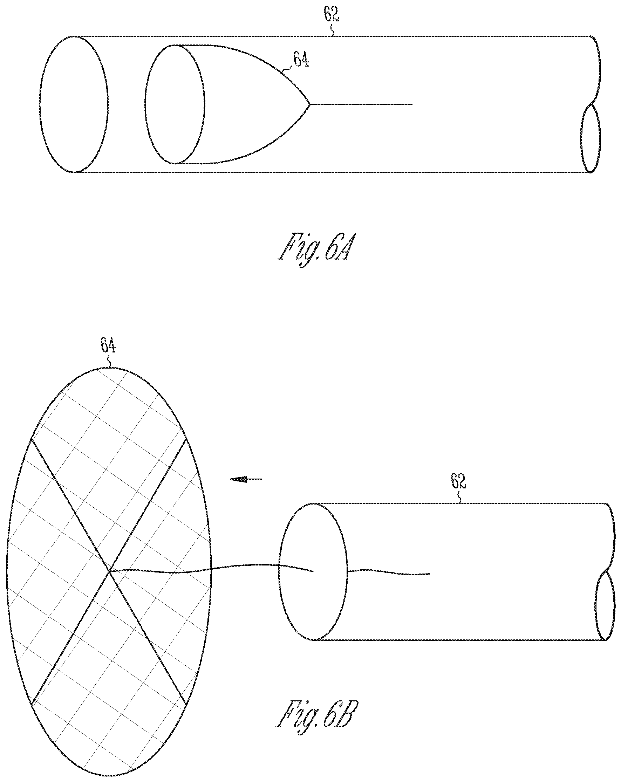

Fig. 6A shows the sealing element 64 constrained by an outer sheath 62 so the sealing element is in a collapsed configuration suitable for delivery to the treatment region.

Fig. 6B shows the sealing element 64 in the expanded configuration after the sheath has been retracted away from it, removing the constraint and allowing the sealing element to self-expand.

Any of the sealing elements may be used with any of left atrial appendage closure devices disclosed herein. Additionally, they may be positioned in any of the locations disclosed herein.

### Anchoring Elements

Any of the anchoring elements disclosed herein may be used in any of the left atrial appendage devices disclosed herein.

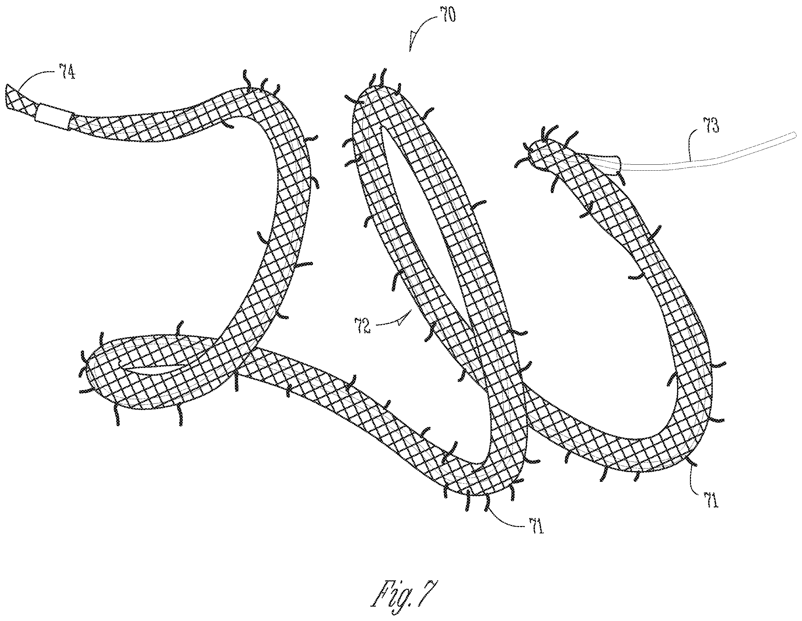

Fig. 7 illustrates an example of an anchoring element 70. The anchoring element 70 includes an outer sleeve 72 which serves as a jacket and is disposed over a flexible wire 73. Preferably the flexible wire 73 is a Nitinol self-expanding wire having a pre-set shape consisting of a plurality of coils or loops that allow the anchor to self-expand and self-contour to various anatomies of left atrial appendage. The coils may be in the same plane, or in disposed in a plurality of planes that are parallel or transverse to one another. Thus, upon deployment the flexible wire will form coils and the outer sleeve 72 will conform to the shape of the flexible wire and also form coils. The outer sleeve may be a metal, a polymer, or other materials known in the art, and the outer sleeve may be braided or take other forms. Optionally, flexible shape-set barbs 71, preferably Nitinol are disposed on the outer sleeve. The barbs may be overmolded polymer or elastic barbs and these barbs help anchor the anchor element to the tissue in or around the left atrial appendage without causing trauma to the tissue. The barbs are flexible and allow them to expand and collapse as needed, for example during loading and collapsing onto a delivery catheter with an outer sheath constraint. The barbs spring open when the constraint is removed. Optionally, the anchor element includes a radiopaque atraumatic tip 74. The barbs 71 may penetrate tissue to help anchor the device but are sized and shaped to avoid pericardial effusion or tamponade. Anchoring force provided by a single barb may be small, but when a plurality of barbs are used, the anchoring force synergizes and provides adequate force to anchor the entire device in place.

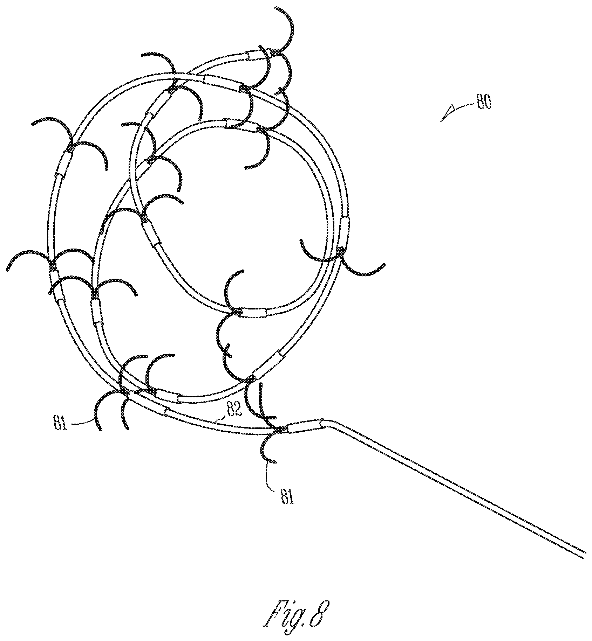

Fig. 8 illustrates another example of an anchor element 80. The anchor element 80 is formed from a filament 82 such as a Nitinol wire which is preferably shape set into a plurality of flexible coils or loops. The coils may be the same size or varying sizes and they may be disposed in the same plane or in a plurality of parallel or transverse planes. This allows the coils to conform to the various sizes and shapes of appendages. A plurality of flexible barbs 81 are coupled to the coils. The barbs may be a shape set metal such as Nitinol or they may be overmolded elastic polymer barbs which allow them to expand and collapse as needed, for example during loading and collapsing onto a delivery catheter with an outer sheath constraint. The barbs spring open when the constraint is removed. Here the barbs are arcuate shaped or C-shaped barbs, although other shapes may be used. The barbs help anchor the anchor element to tissue in or adjacent the left atrial appendage without causing trauma to the tissue.

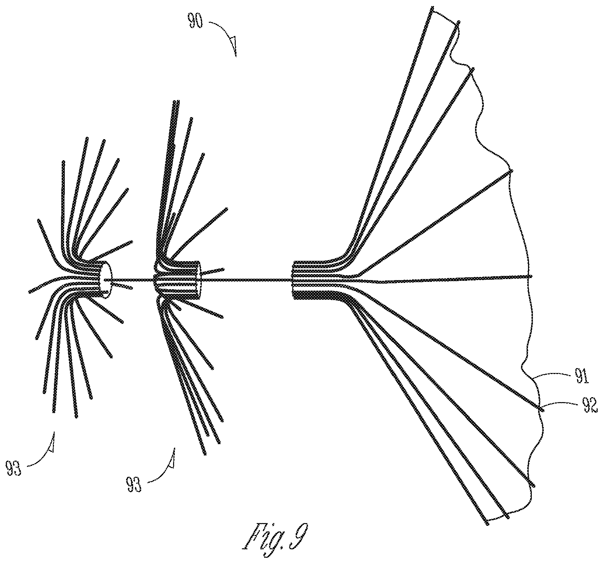

Fig. 9 illustrates an example of a left atrial appendage closure device 90 which includes a sealing element 92 and an anchor element 93. A coating 91 is disposed over at least a portion of the device, such as on the sealing element 92. The coating may be any coating known in the art which facilitates endothelialization of the device, thereby preventing or minimizing thrombosis. In this example, the sealing element 92 includes a plurality of arms extending radially outward from the center of the disc-shaped sealing element forming an umbrella-like structure. These arms form tines to which a cover may be coupled such as PET, ePTFE or other materials known in the art, thereby forming a sealing element which may be positioned in or adjacent to the ostium of the appendage thereby sealing it, and shaped like a circular or disc-shaped flange. Positioning of the sealing element may be in any of the positions disclosed herein. The coating may be disposed on the cover or on the tines.

The anchoring element 93 in this embodiment includes one, two or more discrete anchors. Each anchor is preferably a smaller size than the sealing element and includes a plurality of arms extending radially outward from the center of the anchor. In the embodiment where there are two anchors, each anchor is axially separated a distance away from the adjacent anchor. The plurality of arms form tines which can then engage and anchor to the tissue in or around the appendage. The tines form a circular or disc shaped flange. The anchors may also be coated with any material which helps reduce or eliminate thrombosis. A coupling element, here a wire filament joins the anchor elements to the sealing element forming the closure device. The anchor element is preferably axially separated a distance from the sealing element.

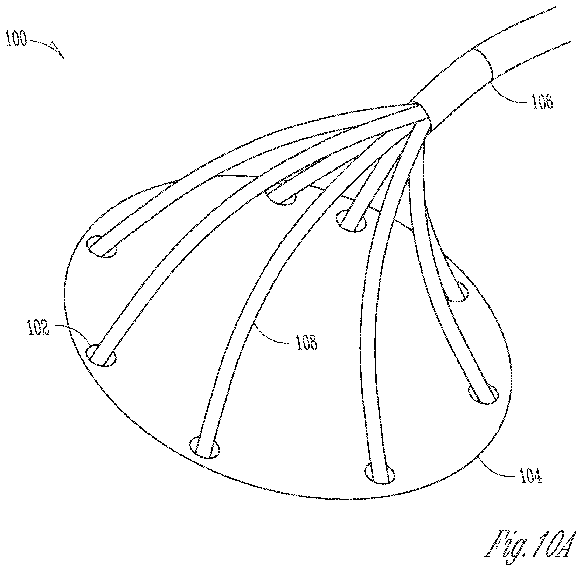

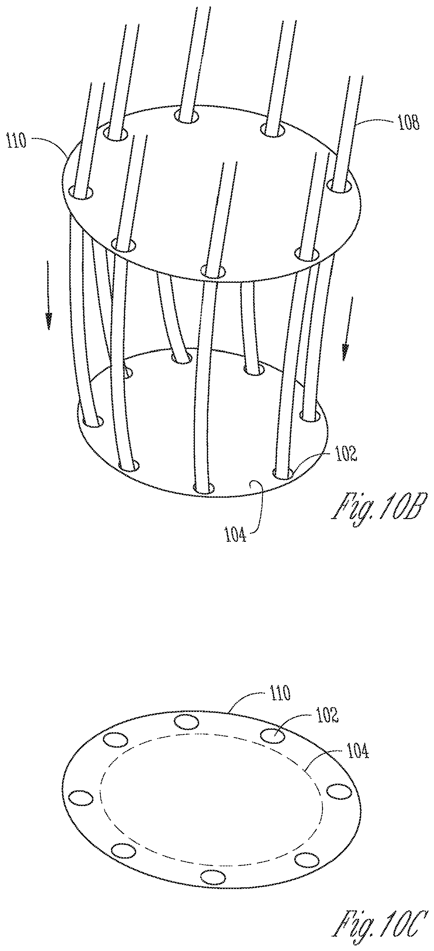

Figs. 10A-10C illustrate another example of a left atrial appendage closure device 100. Fig. 10A shows the closure device still coupled to a delivery catheter 106. The delivery device carries and attaches a plurality of anchor elements 102 into tissue surrounding the perimeter of the ostium to the left atrial appendage 104. The anchor elements may take any number of forms including helical anchors, T-anchors, shape set Nitinol anchors, anchor clips, etc. A filament 108 such as a wire or suture is coupled to each anchor element 102 and serves as a rail over which a cover may be slidably disposed. The cover or other sealing element 110 (best seen in Fig. 10B) is then advanced over the filaments from the delivery catheter into apposition with the tissue surrounding the ostium thereby forming a seal. The cover or sealing element may be any of the sealing elements disclosed herein, such as a fabric, metal, polymer or other lid which seals the ostium. The cover is then secured into position, and it may include any of the coatings disclosed herein that help promote endothelialization to minimize or prevent thrombosis. The delivery catheter may include an inner shaft and an outer sheath for constraining the device during delivery. Retraction of the sheath allows the cover and other elements to expand.

Fig. 10B shows the cover 110 slidably advancing over the filaments toward the ostium of the tissue, and Fig. 10C shows the cover advanced into apposition with the ostium thereby sealing the left atrial appendage.

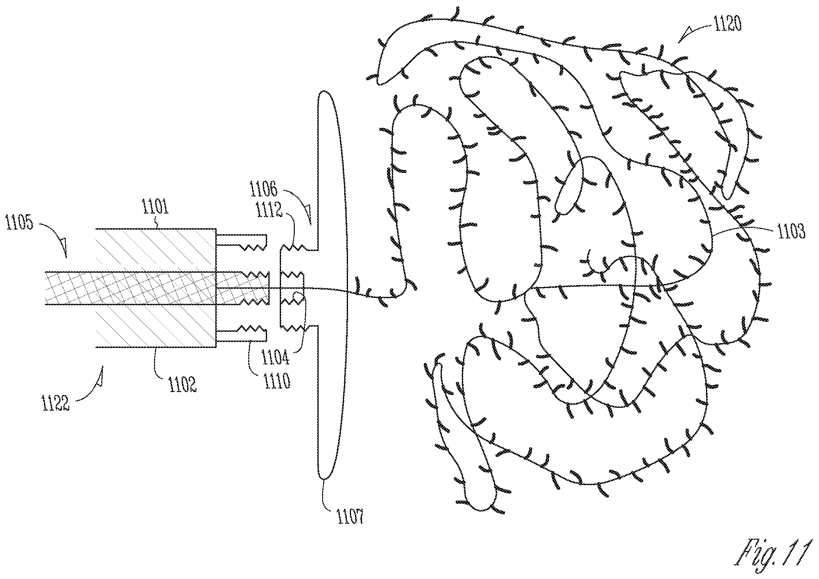

Fig. 11 illustrates an example of a left atrial appendage closure device 1120 and a delivery device 1122 that may be used to deliver the device to the treatment region. The delivery device 1122 includes a delivery catheter 1101 that may optionally include a steering or deflection mechanism for actuating the distal portion of the catheter. The catheter 1101 may include an outer torque shaft 1102 which allows an operator to rotate the catheter to help with positioning during delivery. The outer torque shaft 1102 may optionally include a threaded region 1110 near the distal end which allows the catheter to be threadably coupled to and uncoupled from a thread 1112 on the proximal end of the closure device. The anchor element 1103 may comprise a filament such as a wire (e.g. Nitinol) or suture or other material that is shape set to form a plurality of coils and loops in one or more planes that are parallel or transverse to one another. The filament optionally includes a plurality of barbs disposed on the filament to help the anchor element engage with adjacent tissue without causing trauma to the tissue. A sealing element 1107 is coupled to the anchor element at one end of the filament, thus the filament also serves as a coupling element. The sealing element may be any shape but is preferably a round flat planar disc shaped component for forming a seal with the ostium of the appendage. It may also be a self-expanding component such as previously described in this specification. External threads 1112 on the sealing element allow the closure device to be releasably and threadably coupled to and released from the delivery catheter. The delivery catheter may also include an inner torque shaft 1105 and may preferably have threads which are releasably and threadably coupled or uncoupled with inner threads 1104 on the sealing element. The sealing element and or the anchoring element may be formed from platinum-iridium (Pt-Ir) so that it may be visualized under fluoroscopy. The threading mechanism for coupling the delivery catheter to the closure device may be used with any of the embodiments disclosed herein. One of skill in the art will appreciate that internal threads and external threads may be substituted with one another and therefore are also contemplated as variations on the disclosed embodiments.

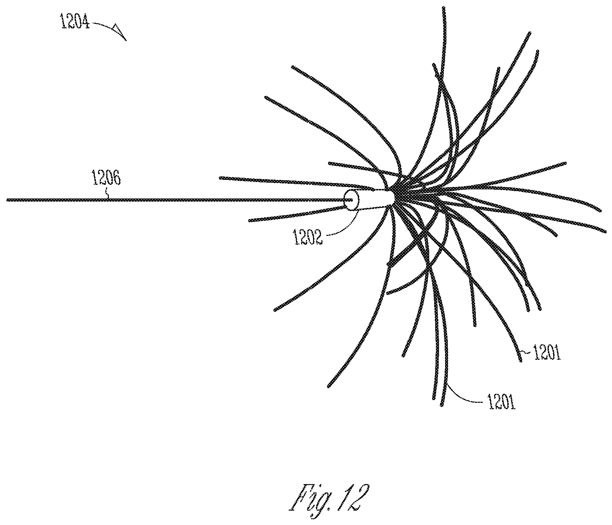

Fig. 12 illustrates another example of an anchor element 1204. The anchor element includes a plurality of filaments such as Nitinol wires extending radially outward from a central hub 1202. The filaments may be shape set to be curved or have any desired shape, and they engage tissue and hold the anchor in position without causing excessive trauma to the tissue. A connector element such as a wire or filament 1206 is coupled to the hub 1208 and allows a sealing element such as any of those disclosed herein to be coupled with the anchor element. Similar to the other embodiments in this specification, the anchor 1204 maybe self-expanding. During delivery it is constrained by an outer sheath into a collapsed configuration and after the sheath is retracted, the constraint is removed and thus the plurality of filaments are free to self-expand into engagement with adjacent tissue in the appendage.

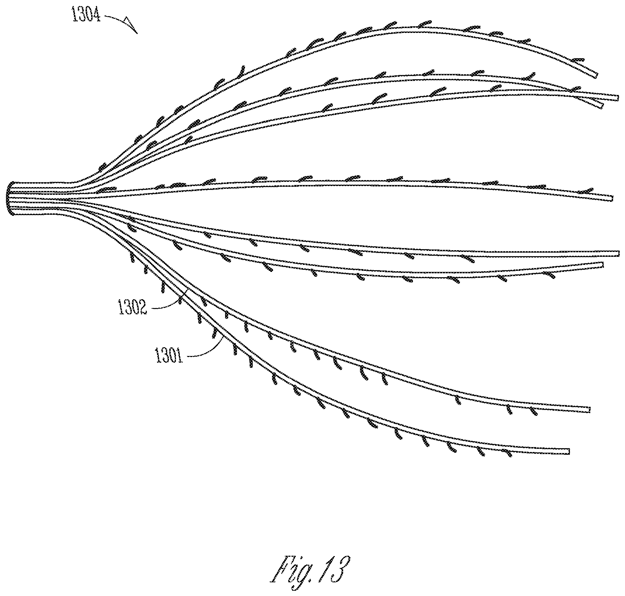

Fig. 13 illustrates another example of an anchor element 1304. The anchor element 1304 includes a plurality of elongate flexible filaments such as wires or coiled elements that are arcuate and extend radially outward from one end of the anchor element 1304. The filaments in this embodiment start out on one end in a relatively flat planar configuration and then bend radially outward in a concave curve, increasing in diameter until an inflection point is reached and then the curve returns inward so that the tips of the filaments are sloping downward and diameter decreases. The filaments are preferably formed from a shape memory alloy such as Nitinol and set to a desired shape so that after a constraint is removed during delivery, the filaments self-expand radially outward into the desired shape where they engage with and anchor to adjacent tissue. The filaments may be the same length or different lengths. Optionally, barbs 1301 are coupled to the filaments to help engage and secure the anchor element to the tissue. The barbs may be metal components coupled to the filament or they may be overmolded elastic polymer barbs coupled to the filaments. The barbs are flexible so they may be collapsed during loading and delivery on a delivery catheter with a constraining sheath, and self-expanding to spring open. Any of the sealing elements, delivery catheters, and release mechanisms disclosed herein may be used with this closure device or any of the other closure devices disclosed herein.

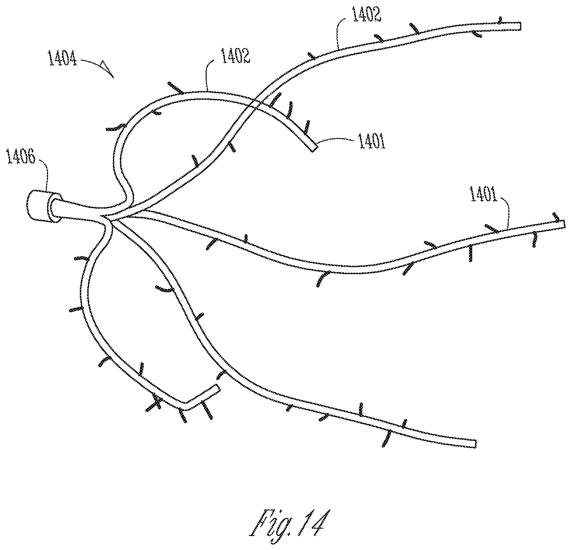

Fig. 14 illustrates another example of an anchor element 1404. The anchor element 1404 includes a plurality of elongate flexible filaments 1402 extending from a central hub 1406. The filaments are preferably Nitinol wires that self-expand into a pre-set shape. The filaments may be various lengths and diameters and may have any number of shapes or curvatures. The filaments 1402 may include a plurality of flexible barbs 1401 for engaging tissue atraumatically. The barbs may be shape set Nitinol barbs or they maybe overmolded polymer elastic barbs that flex during collapsing and constraining with a sheath and self-expand when the sheath is retracted. The hub 1406 may employ the threading mechanism previously described or any other mechanism to allow coupling and uncoupling from a delivery catheter.

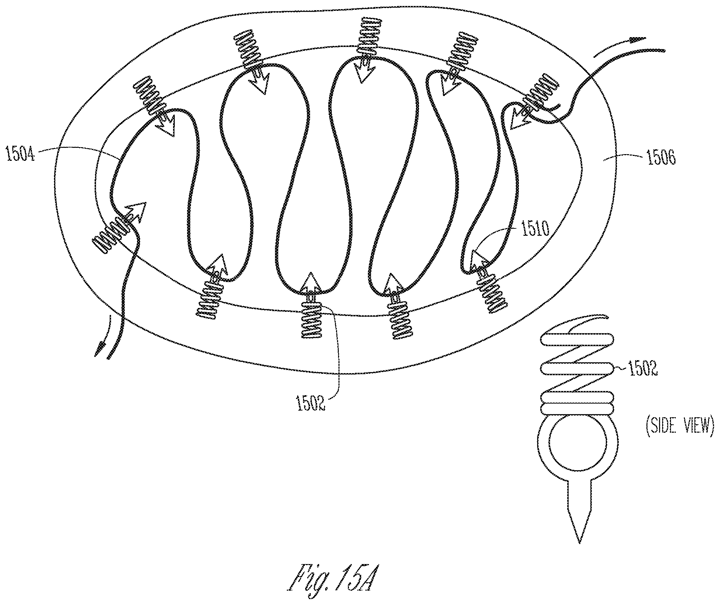

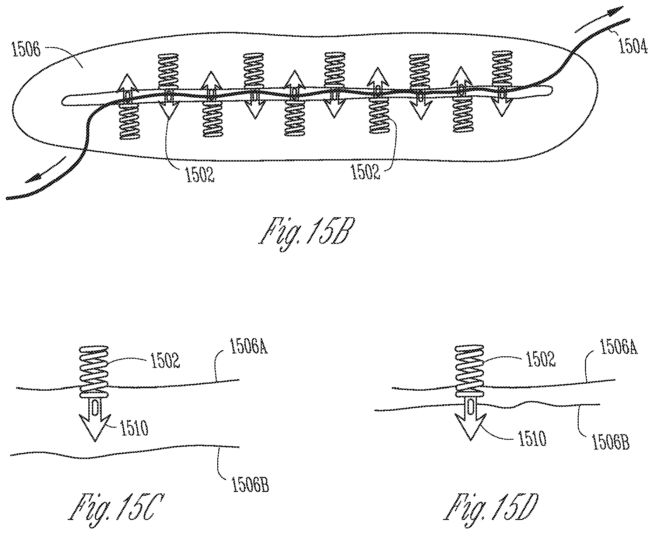

Figs. 15A-15D illustrate another exemplary appendage closure device. In Fig. 15A, the device includes a plurality of anchor elements 1502 secured to one side of ostium (roof) and also the other side of the ostium 1506 (floor). One or more filaments 1504 are coupled to the anchor elements in a pattern similar to a purse string suture pattern. When the ends of the filaments are pulled, the mouth of the ostium closes as seen in Fig. 15B. Tension may be applied until the ostium completely closes and creates a seal to prevent thrombus from embolizing. Fig. 15C illustrates an exemplary anchor element 1502 disposed in tissue 1506a on one side of the ostium. The anchor includes an anchor portion for securing the anchor to tissue 1506a on the one side of the ostium. Barbs 1510 or other engagement elements extend outward such that when the filaments are tensioned, the barbs 1510 pass through tissue on the opposite side of ostium, thereby further sealing the two sides of the ostium together as seen in Fig. 15C.

### Delivery Method

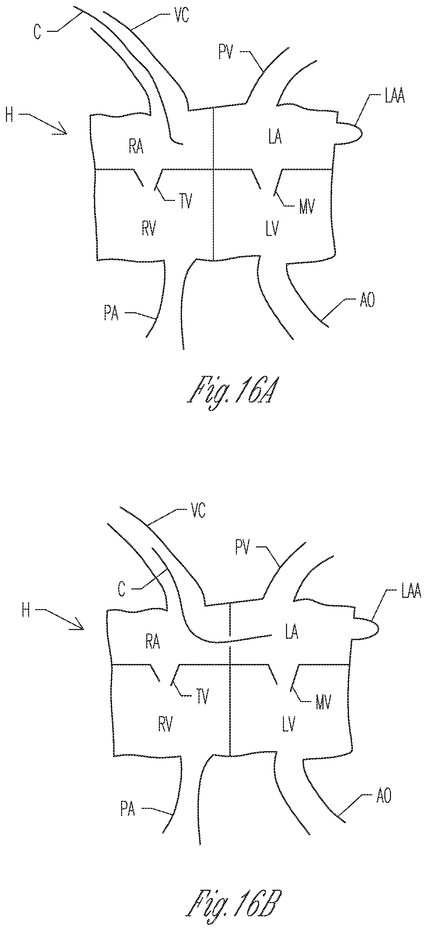

Figs. 16A-16F illustrate an exemplary method of delivering a closure device to the left atrial appendage that may be used with any of the closure devices disclosed herein. Any of the closure devices disclosed herein my be delivered using these methods although one of skill in the art will appreciate that some modification of the methods may be required depending on the specific closure device and delivery system used.

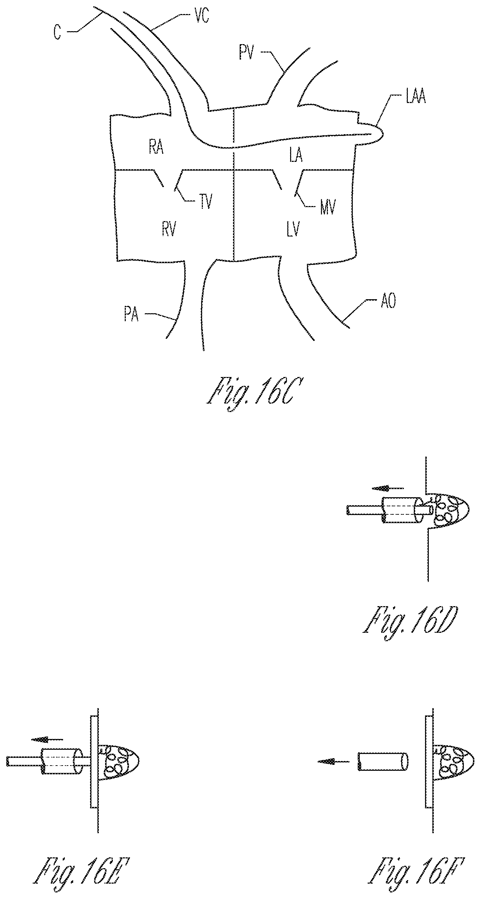

In Fig. 16A basic heart H anatomy is illustrated with a right atrium RA, right ventricle RV, left atrium LA, and left ventricle LV. The tricuspid valve TV controls flow between the right atrium and right ventricle and the mitral valve MV controls flow between the left atrium and the left ventricle. The vena cava VC returns blood from the body to the right atrium RA while the pulmonary artery PA delivers blood from the heart to the lungs for oxygenation. The pulmonary vein PV returns oxygenated blood from the lungs to the left atrium LA, and the aorta delivers blood from the left ventricle LV to the body. A delivery catheter C is preferably percutaneously introduced into a femoral vein in the patient's groin using the Seldinger technique or by cutdown and then advanced over a guidewire (not shown) to the right atrium. The catheter is then delivered transseptally across the atrial septum into the left atrium LA as seen in Fig. 16B. The catheter is then further advanced into the left atrial appendage LAA as seen in Fig. 16C.

In Fig. 16D the outer sheath of the delivery catheter is retracted thereby releasing a constraint from the anchor element and allowing it to self-expand into engagement with tissue in the left atrial appendage. Further retraction of the outer sheath as seen in Fig. 16E then releases the constraint from the sealing element allowing it to self-expand into engagement with the ostium forming a seal. The closure device is then uncoupled from the delivery catheter and left in the patient while the catheter is removed, as illustrated in Fig. 16F.

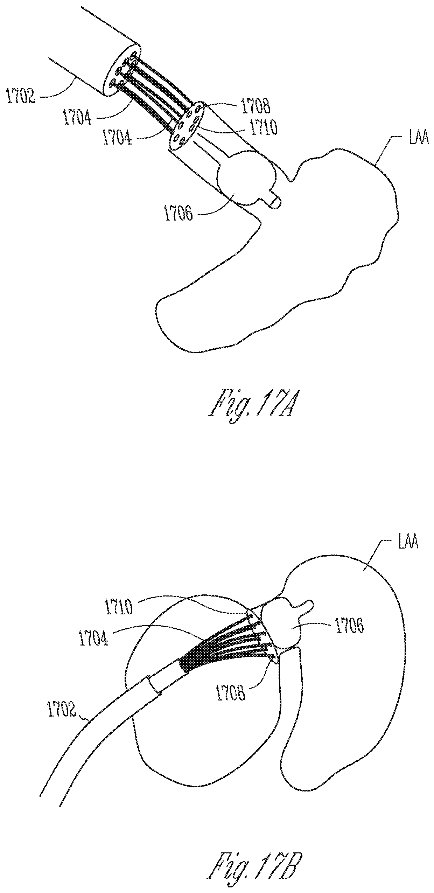

Figs. 17A-17B illustrate another example of a closure device 1708 and a delivery catheter 1702. The delivery catheter 1702 includes a highly compliant, low profile balloon 1706, preferably fabricated from C-Flex, silicone, latex, or another material known in the art, on the distal end of the catheter. The balloon may be inflated in the ostium or in the appendage in order to help center the closure device during deployment. The balloon may be coupled to a catheter shaft that is slidably engaged with a lumen in the outer catheter shaft. The closure device 1708 may take any form but in this embodiment is a coated membrane coupled to a self-expanding, shape memory frame that self-expands to cover the ostium. The coating may be any of the coatings disclosed herein, and the frame membrane may be any of the fabrics or other materials described herein such as PET, ePFTE, etc. Anchors 1710 are coupled to the closure device and are advanced by the delivery catheter into engagement with tissue surrounding the ostium so that they engage the tissue. The anchors may be helical anchors that threadably engage the tissue. Filaments 1704 such as wires or sutures extending axially from the distal end of the delivery catheter act as a guide or a rail so that the anchors can be directed and attached to the tissue. The filaments are slidably disposed in a plurality of lumens in the delivery catheter.

Fig. 17B shows the closure device sealed to the ostium and the balloon expanded to center the device. After the device has been deployed, the filaments 1704 are retracted and removed. The balloon is also deflated and balloon catheter shaft is detached.

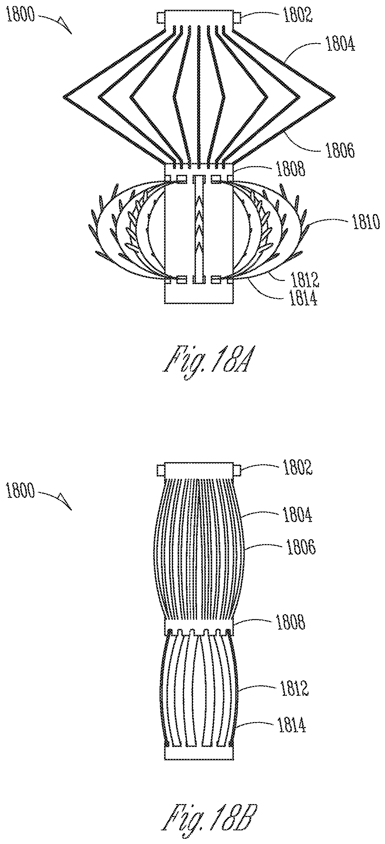

Fig. 18A illustrates another example of a left atrial appendage closure device 1800 in the expanded configuration. The device 1800 includes a proximal portion also referred to as a sealing portion or sealing disc 1804, a distal portion also referred to as an anchor portion or anchor element 1812, and a connector portion or coupling portion 1808 which joins the proximal and distal portions together.

The proximal or sealing portion 1804 is formed from a plurality of elongate axially oriented arms 1806 which are flat and parallel with the longitudinal axis of the device during delivery and radially expanded outward to form a diamond shaped (from a side view) disc, or a diaphragm shaped disc, or accordion shaped disc or cap in the expanded configuration. The proximal portion may be laser cut from hypodermic needle tubing or other known techniques such as photoetching or EDM machining may be used. The arms may be formed from Nitinol or other material so they self-expand into the desired shape upon delivery and once a constraint is released from the arms, or the arms may bend into the desired shape when a compressive force is applied to the arms. The diamond-shaped cap includes upper and lower portions which includes substantially linear arms that meet around the middle portion of the diamond in a point to form the largest diameter of the cap. The proximal ends of the arms are coupled to a ring with a pin or other protuberance 1802 which allows the device to be coupled with a delivery system. Other fastening mechanism besides a pin or protuberance may be used. The distal portion of the arms are also coupled to a ring or collar which forms a part of the connector element 1808. The sealing portion may be covered or otherwise coating with a material to help form a seal at the ostium of the LAA. The material may be polyesther, ePTFE, or any other material known in the art.

The distal or anchor portion 1812 similarly is formed from a plurality of arms 1814 which are flat and parallel with the longitudinal axis of the device during delivery and then radially expanded outward upon delivery to form a basket or distal cage. The arms 1814 include a plurality of barbs 1810 on each arm that help anchor each arm and therefore the distal portion into tissue in the left atrial appendage. When the arms are radially expanded to form the basket, the barbs will also expand radially outward to expose the sharp end of the barb allowing it to engage with tissue and anchor the device. The barbs are oriented to face upward toward the ostium so as to prevent the device from ejecting from the appendage. The arms may also be formed similarly as the arms in the sealing portion and may self-expand or radially expand when a force is applied to the arms. The proximal ends of the anchor arms are also coupled to a ring or collar (not seen in this view) and the distal ends of the anchor arms are free ends and coupled to the distal portion of the sealing portion as will be discussed in further detail below.

Fig. 18B illustrates the device 1800 of Fig. 18A in the collapsed configuration which is used during delivery through the vascular system. Fig. 18B more clearly shows the distal ends of the arms 1814 which are free ends, are disposed in slots in a distal portion of the sealing portion while the proximal arms of the anchor portion are received in slots in a more proximal portion of the sealing portion. The proximal portion of the anchor arms are not visible in this view but terminate in a collar or ring. The distal portion of the arms in the sealing portion terminate in a collar or ring and the proximal portion of the arms in the sealing portion also terminate in a collar or ring.

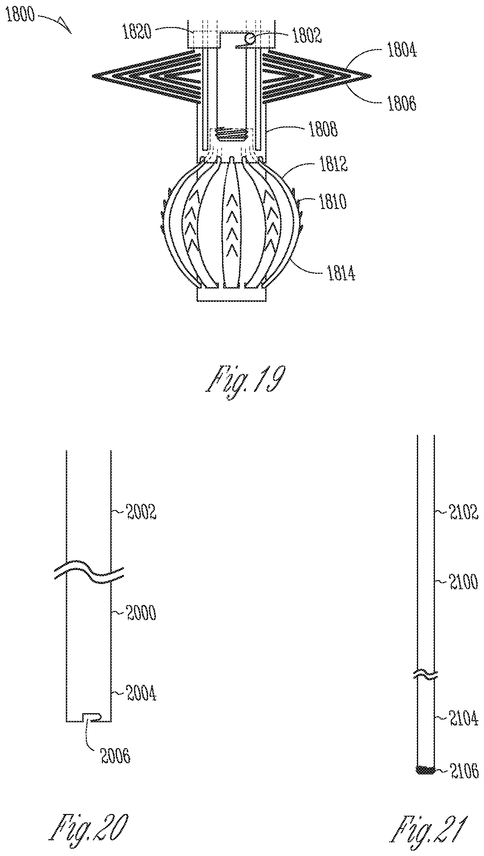

Fig. 19 illustrates the device 1800 in Figs. 18A-18B above coupled to a delivery system which will be described in greater detail below. Both the sealing portion 1804 and the anchor portion 1814 are in the expanded configuration.

Fig. 20 illustrates an elongate shaft 2000 having a proximal portion 2002 and a distal portion 2004 which may form a portion of the delivery catheter. The elongate shaft includes a coupling mechanism such as a bayonet lock 2006 that is configured to engage with the pin or protrusion 1802 or other coupling mechanism on the device 1800. The elongate shaft may be rigid or flexible. Optionally in any embodiment the shaft is a torque cable which allows a user to deflect the delivery catheter to accommodate various LAA anatomies. Further details regarding shaft 2000 are disclosed below.

Fig. 21 illustrates another elongate shaft 2100 having a proximal end 2102 and a distal end 2104 that forms a part of the delivery system. In this embodiment the shaft 2100 is used as the inner-most shaft of the delivery system and the distal portion includes a coupling mechanism 2106, here a threaded region for releasable coupling with the device. In this embodiment, the threaded region on the inner-most shaft is threadably engaged with an inner threaded region on the proximal collar or ring of the anchoring element. One of skill in the art will appreciate that inner and outer threaded regions may be replaced with one another. The shaft 2100 may also be a flexible torque cable. Pushing and pulling the shaft 2100 allows the operator to engage the barbs on the anchor element precisely at the desired location of the LAA anatomy. Also, the delivery system allows the operator to reposition if optimal tissue engagement is not verified on fluoro or echo. Additional details of an exemplary delivery system will be described further below.

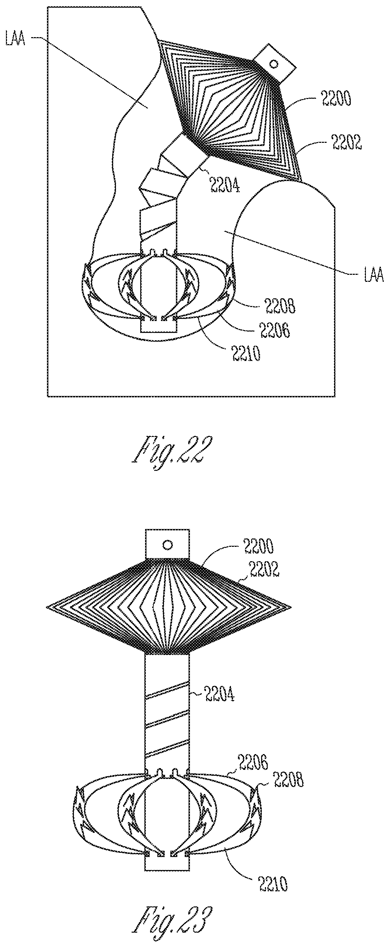

Fig. 22 illustrates a LAA exclusion device 2200 which is a variation on the embodiment of Figs. 18A-18B. The sealing portion 2202 generally takes the same form as previously described above in Figs. 18A-18B. Similarly, the anchor portion 2206 includes a plurality of arms 2210 with barbs 2208 which also generally take the same form as previously described above in Figs. 18A-18B. The coupling element 2204 is the main difference in that rather than a straight or rigid coupling element, the coupling element 2204 in this embodiment is a helical or spiral coil which allows the sealing portion 2202 to flex relative to the anchor portion 2206 which is desirable since this allows the device to accommodate a number of different anatomies of LAA. The helical or spiral cut also allows self-centering of the device in the LAA. This feature allows the proximal sealing element to conform to any of the LAA anatomies regardless of which angle the distal anchor portion is placed inside the LAA. The distal anchor portion can first be placed deep inside the LAA anatomy via the deflectable sheath, and when the proximal sealing portion or disc is unsheathed, the sealing portion can conform to contours of the ostium of the LAA independent of which angle the sealing portion sits relative to the distal anchor. This feature also allows the operator to change the relative position of the anchor portion with respect to a proximal sealing portion or disc that seals the ostium of LAA. The spring-like spiral feature can be stretched to accommodate a range of distance between the anchor and the proximal disc within reason.

Fig. 23 illustrates the embodiment in Fig. 22 with the coupling element 2204 in a straight configuration.

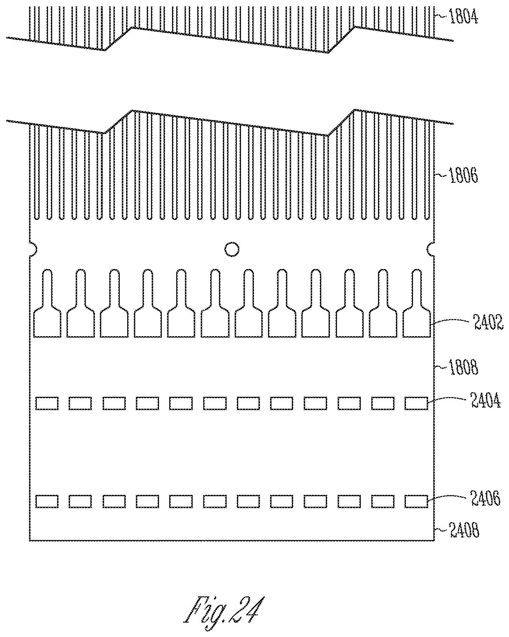

Fig. 24 shows a flat pattern of hypo tube after it has been laser cut to form the proximal or sealing portion of the LAA device in Figs. 18A-18B. The proximal part of the sealing portion 1804 includes a plurality of arms 1806. The arms are thin linear struts that are parallel with the longitudinal axis of the device. The proximal-most portion of the arms is not illustrated in this figure, but the arms are coupled with a solid band of metal which forms the annular ring or collar at the proximal end of the sealing portion. The distal ends of the arms are coupled with a solid band of metal which forms the connector or coupling element 1808 and also the distal collar or ring 2408 of the sealing portion. As previously discussed, the arms may be biased to expand radially outward to form the diamond shaped sealing portion when a constraint is released from the arms. Additionally two rows of slots 2404, 2406 may also be cut into the hypotubing although only one row is needed. The slots are sized to receive the free ends of the anchor arms and secure them into position (seen in Fig. 23). These slots can be any shape but here re rectangular shaped slots. Another row of slots 2402 are also machined in the hypotubing of the sealing portion and they are shaped to have a square or rectangular base with a thin and elongate slot extending therefrom. This is row of slots allows the proximal ends of the anchor arms to be retracted into or advanced from the slots and the shape not only accommodates the arms but also allows the pointed portion of the barbs to be easily received in the slots during delivery when the arms are collapsed flat, and the slots also allow the arms to be easily deployed through the slots during radial expansion.

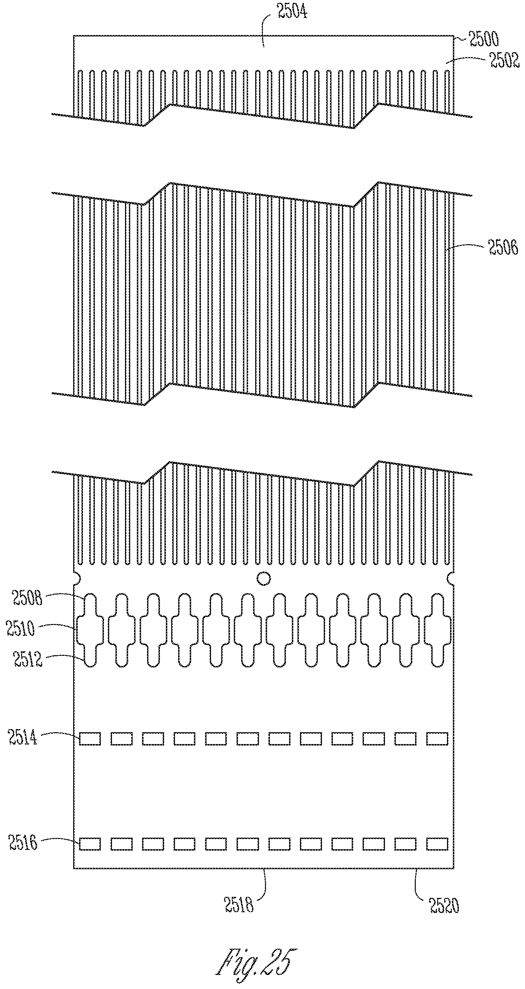

Fig. 25 illustrates an alternative embodiment of a flat pattern of the proximal portion or sealing element 2500 of an LAA exclusion device which may be used in any of the embodiment of LAA exclusion devices disclosed herein. The sealing element is laser cut from hypotubing or fabricated using other known techniques and has a proximal end 2502 furthest away from the LAA ostium and a distal end 2518 closest to the ostium. The proximal and distal ends are formed from solid metal and therefore form a cylindrical band or ring or collar 2504, 2520 at either end of the sealing element. A plurality of elongate axial struts or arms 2506 extend between the proximal and distal rings 2504, 2520. The axial struts 2506 are generally linear struts extending parallel to the longitudinal axis of the device. They are heat shaped to have a radially expanded configuration forming the diamond pattern describe above and biased to expand into the radially expanded configuration when a constraint is removed from the arms 2506. The ends of the arms are coupled to either the proximal or distal rings 2504, 2520.

Distal to the arms are a plurality of slots that have a wide central section 2510 and a narrower and elongate proximal and distal slot 2808, 2512 that extends axially along the sealing portion and generally parallel to the longitudinal axis of the device. The wide central section 2510 allows the arms of the anchoring portion to either slide into our slide out of the slot 2510 during deployment or retraction and the narrower proximal and distal portion 2508, 2512 similarly allow the barbed portion of the anchor arms to slide in or out of the slots during deployment or retraction without binding. Here, the slots are aligned in a one-dimensional linear array to form a single row. The number of slots may match the number of arms in the anchor portion.

Distal to the anchor arms slots are two rows of slots 2514, 2516. Only one row is needed and therefore the proximal-most row of slots 2514 is optional. The distal-most row of slots form linear array of slots in a single row. Each slot is rectangularly shaped and sized and shaped to receive the distal end of the arms in the anchor portion. The distal ends of the arms in the anchor portion include tabs or other features which can lock into position when inserted into the distal slots 2516 thereby securing the distal ends of the anchor arms.

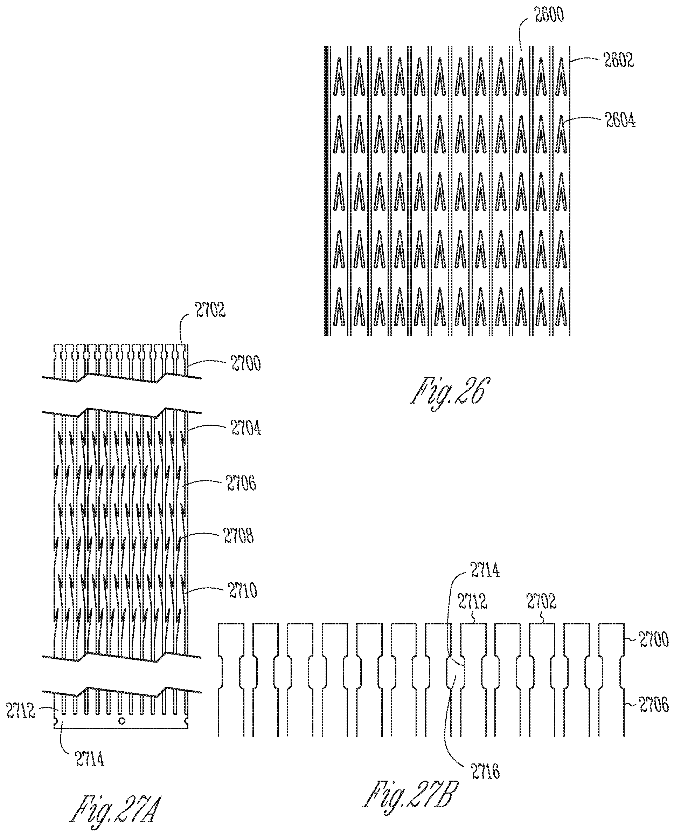

Fig. 26 illustrates a portion of the distal arms 2602 in a distal anchoring portion 2600 of an LAA exclusion device. There are a plurality of arms 2602 which are generally thin narrow elongate struts which extend axially and generally parallel with the longitudinal axis of the device. The arms preferably are connected to a ring or collar at the proximal end (not shown) and the distal portion of the arms are free ends which are inserted into slots 2514 or 2516 and anchored. The arms 2602 may also be heat shaped to have a biased configuration that they self-expand into when a constraint is removed therefrom. Each arm 2602 includes a plurality of barbs 2604 with a pointed tip that engages and helps anchor the arm into the tissue of the LAA. Here, only a single barb is disposed across the width of a single barb but a plurality of barbs are disposed along the longitudinal axis of the arm. This results in a two-dimension array of linear rows and linear columns of barbs on the plurality of anchor arms that are aligned with one another but may be staggered or have any other pattern. And as discussed above, the arms are slidably disposed in the slot 2510 and the barbs pass through slots 2508 or 2512 without binding.

Fig. 27A illustrates a flat pattern showing a variation on the embodiment of the anchor portion 2700 of the LAA device similar to that in Fig. 26 with the major difference being the barbs 2708, 2710. The anchor portion 2700 may be used in any of the embodiments of LAA devices and includes a distal end 2704 and a proximal end 2712 and a plurality of arms 2706 extending therebetween. The arms are elongate linear struts extending axially and generally parallel to the longitudinal axis of the device. Alternating rows of barbs 2708, 2710 are on either the left or right side of the arms and face toward the proximal portion in order to provide anchoring with tissue in the LAA. The proximal ends of the arms are coupled to a ring or collar 2714 while the distal ends of the arms are free ends having engagement tabs 2702 for engagement with slots in the sealing portion.

Fig. 27B highlights the free ends of arms 2706 in anchor portion 2700. The engagement tabs 2702 include an enlarged head region 2712 coupled to a narrower neck region 2714 that is joined to the remainder of the arm 2706. The narrow neck region 2714 forms a slotted region 2716 between adjacent free ends and this allows the distal ends of the arms to lock with their mating slot in the distal part of the sealing portion. This configuration may be employed in any of the anchor portions. Other aspects of Figs. 27A-27B generally take the same form as other anchoring portions described herein.

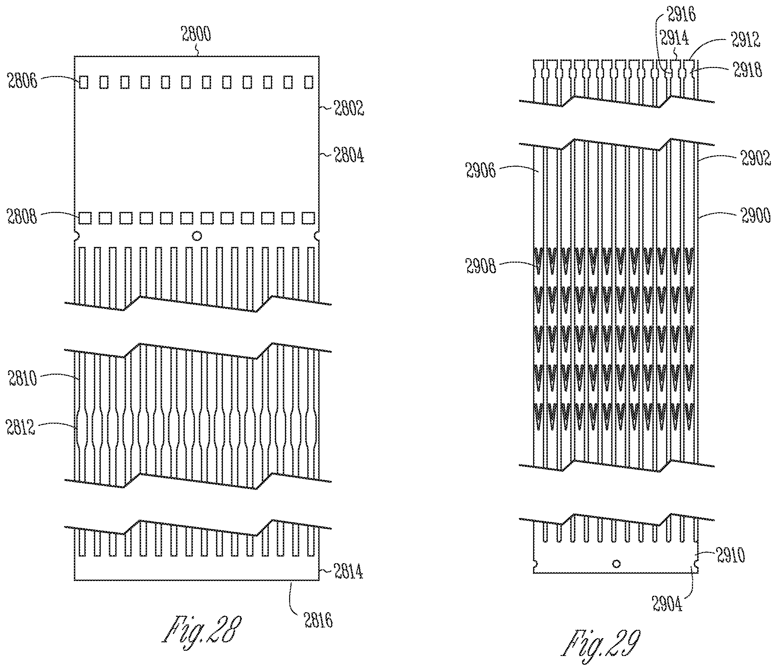

Fig. 28 illustrates a flat pattern of the sealing portion 2800 of the LAA device which may be used in any embodiment. The sealing portion 2800 includes a distal end 2802 and a proximal end 2816. The proximal and distal ends 2816, 2802 form collars or rings 2814, 2804 at either end of the sealing portion 2800. A plurality of radially expandable arms 2810 extend between the rings or collars 2804 and each end of an arm is coupled to a proximal and distal ring 2804, 2814. The arms are substantially linear and extend parallel to the longitudinal axis of the sealing portion 28100 and include a narrow proximal and distal section coupled together with a wider connecting section 2812 which is where a bend may be formed between the proximal and distal portions of the arms. As previously discussed the arms may be heat shaped to form a diamond shaped radially expanded configuration to which the arms self-expand into when a constraint is removed therefrom. Adjacent the distal ends are first and second rows of slots 2806, 2808. While only one row of slots is used, the second row is optional. The slots are aligned in a single row of rectangular or square shaped slots sized and shaped to receive the distal tab ends of the arms in the anchor portion of the device. The tabs lock in the slots thereby avoiding the need for welding, adhesives or other bonding techniques for coupling the two elements together. Other aspects of Fig. 28 generally take the same form as other embodiments of the sealing portion described in this specification.

Fig. 29 illustrates an example of a distal anchoring portion 2900 of a LAA device shown in a flat pattern, and it includes a proximal end 2904 and a distal end 2902. In The proximal end 2904 includes a ring or collar 2910 to which a plurality of arms 2906 are coupled. The distal part of the arms are free ends with the connecting tabs 2912 having an enlarged head portion 2914 and a narrow neck region 2918 that forms a slotted region 2916 between adjacent tabs. The tabs allow the free ends of the arms to be inserted into the slots on the distal end of the sealing portion and anchored there as previously discussed. Barbs 2908 are disposed along the elongate struts or arms 2906 that extend parallel to the longitudinal axis of the anchoring portion. Here the barbs are a single linear array of barbs that are also parallel with the longitudinal axis and point proximally. This embodiment is substantially the same as that of Fig. 26 and maybe used in any embodiment of an anchoring portion of the device. Other aspects of the anchoring portion are generally the same as previously disclosed above.

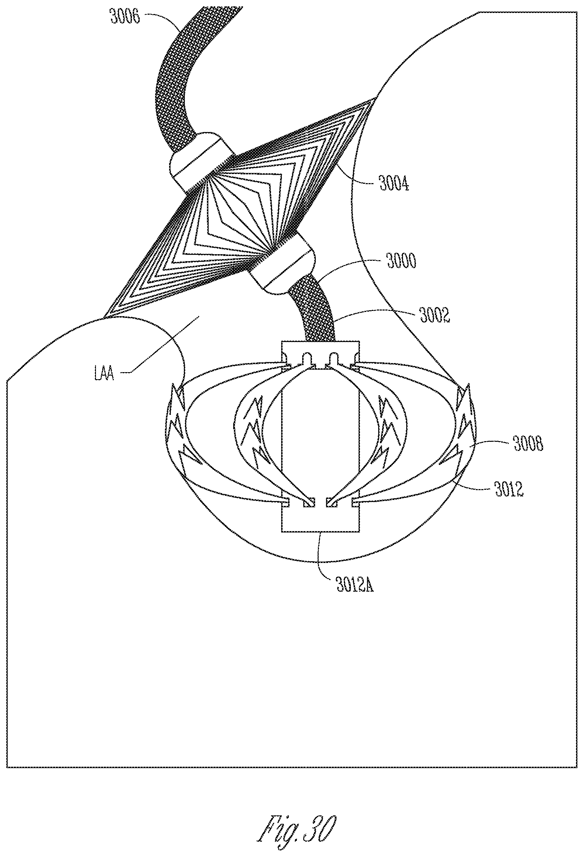

Fig. 30 illustrates another example of a LAA exclusion device 3000. The device 3000 includes a sealing portion 3004, an anchor portion 3012 and a connector element or coupling element 3002 joining the sealing portion with the anchoring portion. The sealing portion 3004 generally takes the same form as any of the other sealing portions disclosed in this section, and similarly the anchoring portion which includes a plurality of arms 3012 with barbs 3008 also generally takes the same form as any of the other anchoring portions disclosed herein. A proximal tether 3006 may be coupled to the proximal end of the sealing portion 3004. The anchor portion may be deployed in the LAA first by retraction of an outer sheath. The sealing portion may be deployed next by delivering it over tether 3006 to mate with the connector element 3002, self-center with the LAA and lock in position thereby sealing the LAA. The tether 3006 may then be released from the sealing portion via a coupling mechanism such as a frangible connection or by using an electrical current to sever the tether.

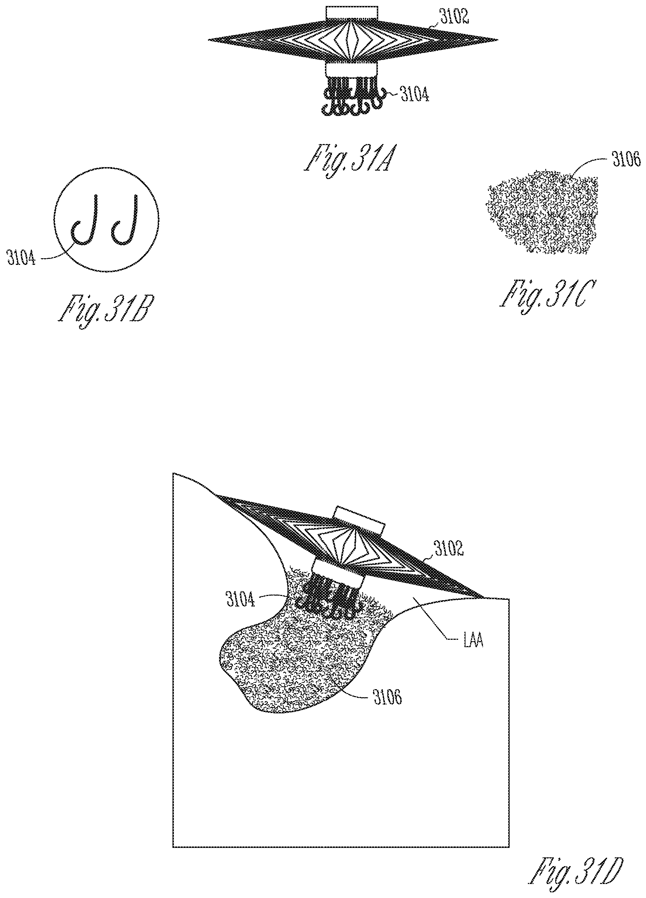

Fig. 31A illustrates another example of a sealing portion 3102 with a coupling mechanism such as hooks 3104 which may be coupled to an anchor portion 3106 seen in Fig. 31C similar to VELCRO or other hook and loop coupling mechanisms. Fig. 31B highlights the hooks 3104 on the sealing portion 3102 of the device. The loop portion may be delivered to the LAA and used to fill the space, then the sealing portion is advanced toward the LAA so that the hooks 3104 engage with the loops 3106 to secure the two portions together. Fig. 31D shows the sealing portion engaged against the ostium of the LAA with anchor portion disposed in the LAA and the hook and loop system coupling the two portions together. The anchoring loop 3106 is a 3D shaped series of loops and coils with tiny barbs similar to the ones described in Fig.7 above. The plurality of the barbs in the coil anchor the loop part 3106 of this device to the walls of the LAA.

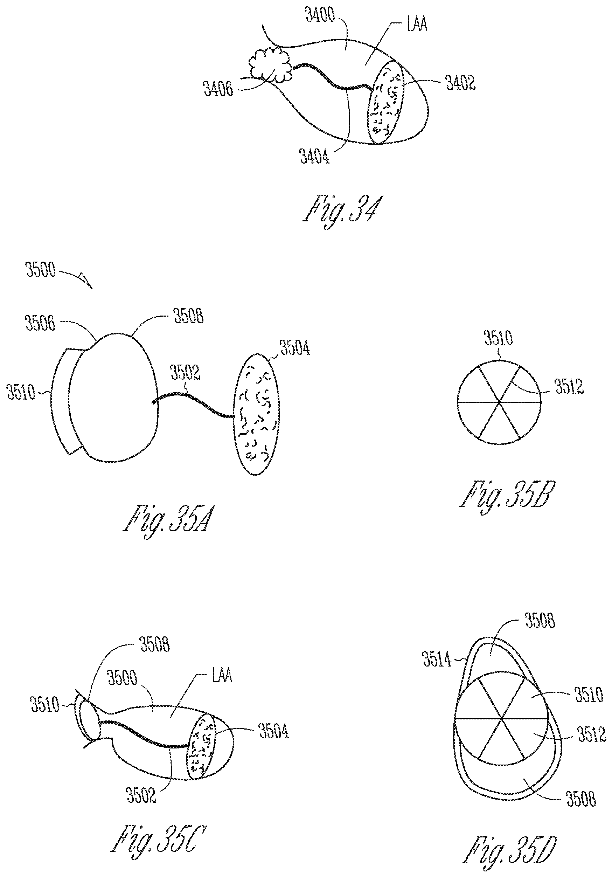

Fig. 34 shows a proximal sealing system 3400 that may be tethered or otherwise coupled to anyone of the anchoring mechanism described herein. The proximal seal 3406 may be an expandable polymer or sponge coated with the peptide or it may remain uncoated. It may be delivered in a collapsed configuration and then expanded when positioned. The expandable polymer will complete a seal against the LAA without any gap, thereby excluding the LAA. A tether 3404 may be used to couple the proximal seal with the distal anchor element 3402. The tether may be a filament of material, a wire, or any other coupling element. The distal anchor element may be any of the anchors described herein, and it may or may not have barbs.

Figs. 35A-35D illustrate another example of a LAA closure device which may be used to form a complete seal of the LAA. Fig. 35A shows a side view of the LAA closure device 3500 which includes a distal anchor portion 3504, a proximal sealing portion 3506, which has a proximal seal or cap 3510 and a compliant balloon 3508 fused to its rear surface. A tether 3502 or any other coupling element may be used to join the proximal sealing portion 3506 with the distal anchor element 3504. The proximal seal or cap 3510 may include any of the coatings disclosed herein and may also include a cover such as a polymer, fabric or tissue to help form the seal.

Fig. 35B shows an end view of the proximal seal or cap 3510 and shows that it may include a plurality of splines or ribs 3512 to help it self-expand and/or provide rigidity during or after deployment.

Fig. 35C shows the device of Figs 35A-35B implanted in a LAA. The proximal seal or cap 3510 seals against most of the ostia, but gaps may exist and therefore the compliant balloon 3508 may be expanded with a fluid such as saline, contrast media or any other fluid to expand the balloon and fill the gaps thereby forming a seal at the ostia. Tether 3502 joins the proximal seal with the anchor element 3504, which may be any of the anchor elements disclosed herein. The anchor element may be coated with any of the coatings disclosed herein.

Fig. 35D shows a front-end view of the sealing cap 3510 disposed against the ostia with gaps due to the asymmetry of the ostia. The balloon 3508 once expanded fills the gaps and provides a seal. Optional ribs or splines 3512 in the sealing cap may help the cap to self-expand or provide rigidity.

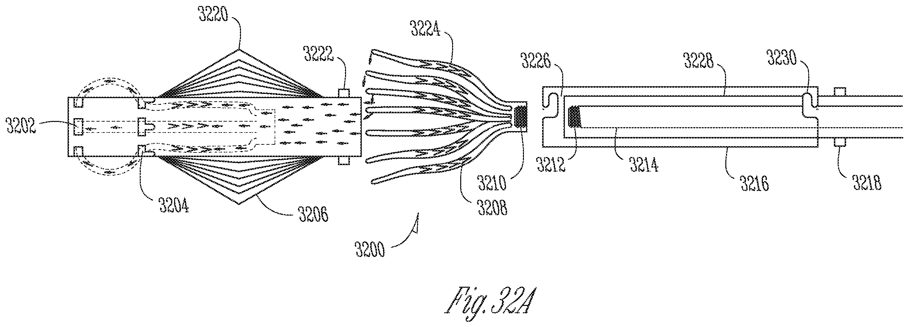

Fig. 32A illustrates a partially exploded view of a LAA closure device and delivery system 3200. The system includes a proximal sealing portion 3220 having a plurality of splines or arms 3206, a distal anchor portion 3224 with barbs on the arms 3208, and three shafts of a delivery system including a deployment guide 3228, a proximal seal control shaft 3216 and a anchor push/pull shaft 3214. A fourth outer sheath is disposed over the three other shafts but is not illustrated in this view for ease in viewing the individual elements. In use, the free ends of the arms of the distal anchor portion are collapsed and then inserted into the inner lumen of the distal sealing element and advanced distally. The free ends then exit the proximal exit port slots 3204 and then the distal ends are inserted back into the distal port anchor arm locks 3202 which are also slots as discussed above. A pin lock such as a bayonet coupling 3226 on the distal end of the proximal seal control shaft 3216 is releasably coupled with the proximal pin 3222 on the sealing portion 3220, and a corresponding proximal pin lock 3230 is simultaneously lockable and releasable with a pin 3218 on the proximal portion of the deployment guide 3228 Locking both ends anchors the distal end of the proximal seal control shaft 3216 with the sealing portion 3220 and anchors it to the deployment guide shaft 3228 thereby preventing movement. The outer thread 3212 on the anchor push/pull shaft 3214 couples this shaft with the inner threads 3210 on the anchoring portion of the device. Other aspects of the sealing portion or anchoring portion generally may take the same form as any of the sealing or anchoring embodiments disclosed herein.

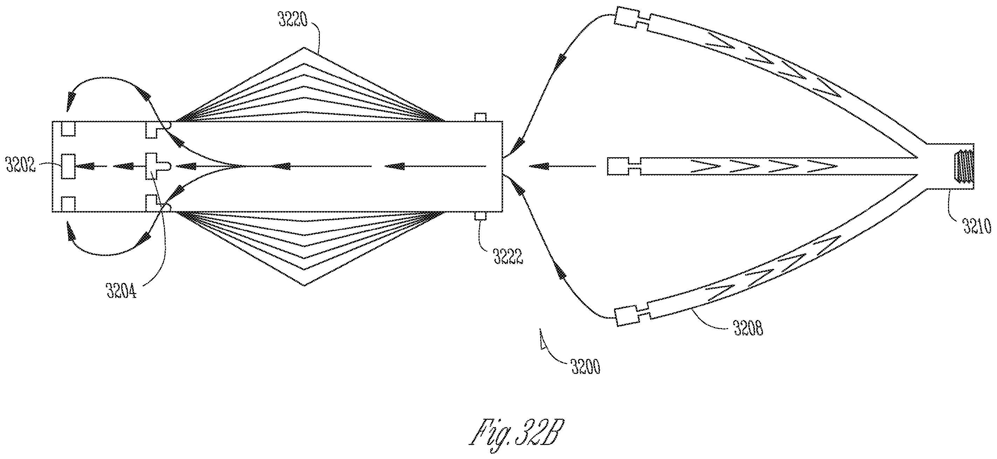

Fig. 32B illustrates assembly of the sealing portion with the anchor portion of the device. The deliver system is not coupled with the device. The anchor arms with barbs 3208 of the anchoring portion are inserted into the lumen of the proximal sealing portion and advanced proximally until they exit the distal slots 3204 in the proximal ring or collar of the sealing portion. The arms then extend over the outer surface of the ring or collar of the sealing portion and then the arms are reinserted into the proximal slots 3202 of the sealing portion ring where the lock in place due to the tabs previously described above. Inner thread 3210 on the anchor portion and proximal pin 3222 on the sealing portion allow the device to be coupled to the delivery system.

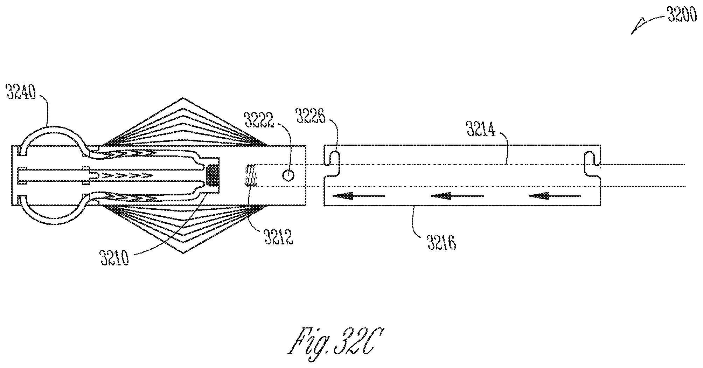

Fig. 32C shows the proximal sealing portion of the device coupled with the distal anchoring portion such that a portion of the anchoring arms 3240 are disposed outside of the collar or ring on the sealing portion and the barbed portion o the arms are disposed underneath the collar of the sealing portion. Once the two halves are assembled together, the threaded regions 3210, 3212 of the anchor portion and the anchor push/pull shaft 3214 may be threadably coupled together. Then the pin lock 3226 on the proximal seal control shaft 3216 may be locked wih the pin 3222.

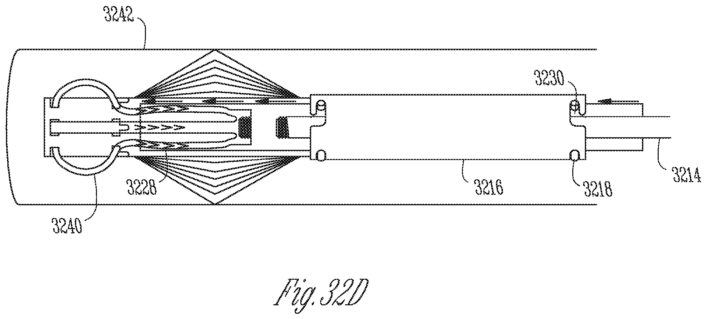

Fig. 32D shows the device fully coupled to an exemplary delivery system. An outer sheath or catheter deployment device 3242 is disposed over all three shafts and also over the LAA closure device such that distal anchor arms 3240 are constrained along with the proximal sealing portion. A deployment guide shaft is also advanced distally in between the proximal seal control shaft and the anchor push/pull shaft and over a portion of the distal anchor until it bottoms out. The proximal seal control shaft 3216 is also locked with pins on the proximal and distal ends and the anchor pull/push shaft is threadably engaged with the anchor portion. The device is ready for delivery using any of the delivery methods described herein (e.g. such as via the transseptal route shown in Figs. 16A-16C. Once the LAA closure device has been delivered to the LAA, it may be deployed.

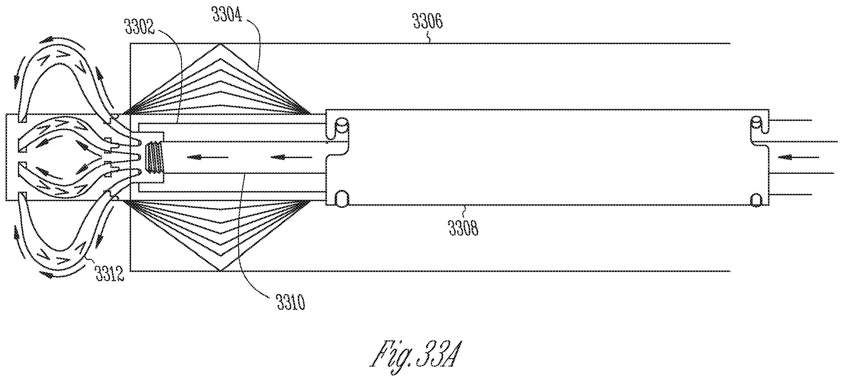

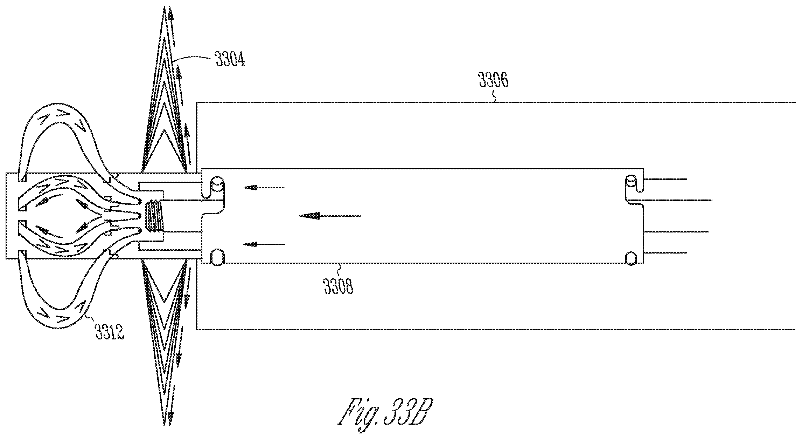

Figs. 33A-33B illustrate exemplary deployment of a closure device using the delivery system of Figs. 32A-32D and may include any of the closure devices described herein. The delivery system may be advanced to the LAA as previously described in Figs. 16A-16F. The delivery system includes an anchor portion 3312 of the device coupled to a sealing portion 3304 of the device. The device is loaded onto a delivery catheter which includes four shafts. The inner-most shaft is the anchor push/pull shaft 3310, the deployment guide shaft 3302 is disposed over the push/pull shaft 3310 and has its distal end abutted against the proximal end of the anchor portion. The proximal seal control shaft 2208 is slidably disposed over the deployment guide 3302 and has bayonet style locks engaged at the distal end with the proximal end of the sealing portion and proximally with the deployment guideshaft 3302. The fourth shaft is an outer sheath or catheter deployment device 3306 slidably disposed over the three other shafts and provides a constraint to self-expansion of the sealing portion or the anchor portion of the device.

Once the delivery system has been advanced to the LAA as described in Figs. 16A-16F, Fig. 32A shows the outer sheath (also referred to herein as the catheter deployment device) 3306 may be partially retracted to expose the arms of the anchor portion 3312 while the sealing portion is still covered by the outer sheath, and also the anchor portion of the device is deployed by pushing distally on the anchor push/pull shaft 3310. Advancement of the anchor push/pull shaft 3310 pushes the arms of the anchor portion out of the proximal-most slots in the distal portion of the sealing portion and since the distal ends of the arms are secured in the distal-most slots of the sealing portion, the arms bow outward thereby radially expanding the anchor arms as illustrated. The bowing also deflects the barbs outward so that the sharp end is exposed and can anchor on tissue. The anchoring portion is then anchored into the LAA.

Fig. 32B shows deployment of the sealing portion. Here outer sheath 3306 is retraced proximally to remove the constraint from the arms of the sealing portion 3304 which then self-expand radially outward to form the diamond-shaped sealing cap.

While not illustrated, deployment may be completed with the following steps. The proximal and distal bayonet locks are then released allowing the proximal end of the sealing portion to move toward the distal end of the sealing portion, thereby further allowing the arms of the sealing portion to self-expand. The second, third, or fourth shafts may also be advanced distally to help move the ends of the sealing portion toward one another. The deployment guide shaft is retracted proximally so it is removed from under the sealing portion and finally the anchor push/pull shaft is threadably decoupled from the sealing portion and the implant is left behind in the LAA while the delivery device is removed from the patient.

One of skill in the art will also appreciate that at any of deployments steps preceeding decoupling of the push/pull shaft from the anchor portion, the delivery device may be actuated in the opposite direction in order to recapture any or all parts of the LAA closure device. This allows and operator to reposition the device if the positioning is incorrect.

In order to deliver the anchor or proximal portion and the seal or distal portion to very complex LAA anatomies, a guidewire-based delivery system may be used. In this case, a guidewire of suitable stiffness and atraumatic tip is first placed deep inside the LAA. The LAA occlude device is then tracked over the guidewire to optimal anchoring spot. Further deployment of the anchor and the proximal sealing disc could be achieved in any one of the ways described above. Thus, the guidewire based delivery system and method may be used with any of the LAA occlusion devices disclosed herein.

### Coatings

All or any portion of the devices and delivery systems disclosed herein may be coated with chemicals or agents that promote endothelialization thereby reducing thrombus formation and reducing the need for anticoagulants after implantation. One or more of the methods described herein includes use of a device or delivery system that is partially or entirely coated with a coating. A coating as described herein is configured to cover (either completely or partially) any surface, either external or internal, of the device and delivery system described herein. Any of the coatings described herein are suitable for covering (either partially or completely) any surface of any of the components described herein. Non-limiting examples of surfaces suitable for coating with the coatings described herein include a surface located on the top of a sealing element, a surface located on the bottom of a sealing element, a surface located on a connecting element, or a surface located on an anchor element. In addition, described herein are methods for making and applying a coating to a device or delivery system as described herein.

The biocompatibility (and/or endothelialization) of a device or delivery system as described herein is significantly enhanced when the surface of the device or delivery system is coated. One type of coating that is suitable for use with the devices and delivery systems described herein is a polypeptide based coating, which, in some embodiments, is covalently bonded to a surface of a device or delivery system. Coating the surface of the device or delivery system promotes endothelialization by, for example, enhancing the migration and proliferation of cells in proximity to the coating, and also provides a bio-friendly surface that promotes the cell's maintaining a normal cell morphology. In contrast, cells growing in hostile or non-biocompatible surfaces tend to deform and are not fully functional.

In some embodiments, a coating comprises a cell binding polypeptide which is configured to promote cell attachment, adhesion and proliferation through the targeting of specific cell membrane receptors such as integrins. Non-limiting examples of polypeptides suitable for use as coatings (components of coatings) for the devices and delivery systems described herein are RGD, found in fibronectin, collagen and vitronectin; REDV, found in fibronectin; and YIGSR, found in laminin. The 15 amino acid-long polypeptide, GTPGPQGIAGQRGVV (P15) is the cell binding site found in collagen.

In some methods for manufacturing a coating, the method comprises modifying a surface of PET, by for example, hydroxylation is carried out and a polypeptide is bonded to the modified PET. Hydroxylation of PET can be carried out by placing a PET sample in 30/70 v/v 1,1,3,3-tetramethylguanidine (TMG)/ethylene glycol (EG) for two hours. The hydroxylated PET is then activated by reacting with disuccinimidyl glutarate (DSG). The activation is carried at room temperate in acetonitrile solution (8 mg of disuccinimidyl glutarate, 24 hours at room temperature). The activated PET is reacted with the polypeptide in pH 7.2 phosphate /NaCl buffer (0.1 M phosphate/0.1 M NaCl) to prepare PET bonded with the polypeptide. The reaction is expressed as a chemical reaction is shown below where "polypeptide" = GTPGPQGIAGQRGVV:

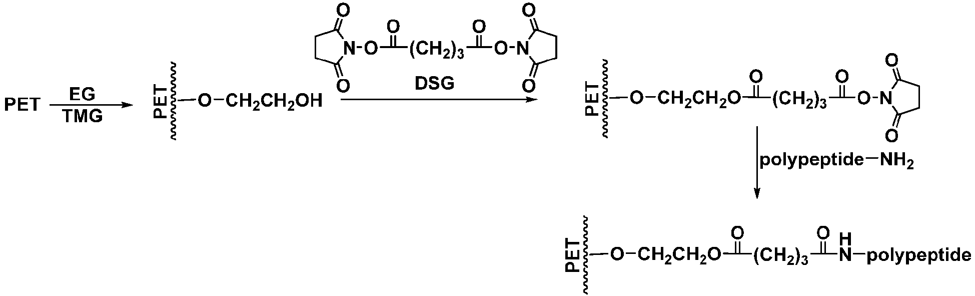

In some methods for manufacturing a coating, the method comprises amination with ethylenediamine (EDA) and bonding a polypeptide. Amination is carried out at varying percentage by volume (10 to 90%) of EDA in ethylene glycol to prepare PET samples with different degrees of aminations. Aminated PET samples are activated by reacting with DSG as in the case of hydroxylated PET. Bonding of the polypeptide is carried by reacting the activated aminated PET with the peptide dissolve in pH 7.2 phosphate buffer. The reaction is expressed as a chemical reaction is shown below where "polypeptide" = GTPGPQGIAGQRGVV:

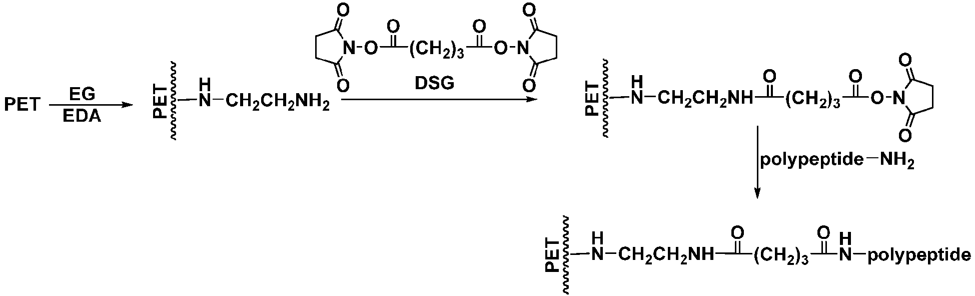

In some methods for manufacturing a coating, the method comprises *IN-SITU* modification of a Left Atrial Appendage Closure (LAAC) device. Using the flushing port of a device as described herein, first the device is fully wetted with phosphate buffered saline. Then a solution of 10-90% EDA may be used (and preferably 80% EDA) is used in ethylene glycol is slowly introduced. The EDA solution is allowed to react for 5 minutes by continuous slow infusion of the reaction mixture. Excess reagent is washed away with repeated flushing with buffer. A solution of disuccinimidyl glutarate in acetonitrile then is infused for 2 minutes. After washing with phosphate buffered saline (PBS), a solution of P15 peptide is infused and allowed stand for 1 hour. Finally, any unreacted peptide is removed by flushing the device with PBS several times.

In some methods for manufacturing a coating, the method comprises surface modification of ePTFE and bonding P15 to modified ePTFE. ePTFE has better hemo and biocompatibility than many other polymers. A LAAC device, in some embodiments, is made with ePTFE. In some embodiments, ePTFE is modified with P15 polypeptide. To increase the wettability of ePTFE first it is treated with atmospheric plasma. This causes the polymer strands to break at random location. This can be achieved with an argon or helium atmospheric plasma system. The plasma treated polymer is reacted with a mixture of sodium hydroxide and chloroacetic acid at room temperature overnight. P15 peptide is then covalently attached to the activated ePTFE using EDC (1-ethyl-3-[3-dimethylamino-propyl] carbodiimide hydrochloride) as a coupling agent. After the peptide is coated, the ePTFE containing devices are vigorously rinsed, for example, six times with deionized water. After a final rinse with absolute alcohol, the devices are air dried.

### NOTES AND EXAMPLES

The following, non-limiting examples, detail certain aspects of the present subject matter to solve the challenges and provide the benefits discussed herein, among others.

The present disclosure generally relates to medical systems, devices and methods, and more particularly relates to left atrial appendage closure devices, systems, and methods.

In a first aspect, a device for sealing a left atrial appendage comprises an anchor element configured to anchor the device to tissue in or adjacent the left atrial appendage, a sealing element configured seal the left atrial appendage and prevent thrombus from embolizing therefrom, and a coupling element joining the anchor element with the sealing element.

The anchor element may comprise barbs, a plurality of tines, or one or more coils.

The anchor element may comprise a plurality of arms with barbs disposed thereon having an expanded configuration for anchoring to tissue in the left atrial appendage, and a collapsed configuration for delivery to the left atrial appendage, and wherein the plurality of arms form an arcuate basket in the expanded configuration.

The sealing element may comprise a plurality of arms having an expanded configuration for sealing the left atrial appendage, and a collapsed configuration for delivery to the left atrial appendage, and wherein the plurality of arms form a diamond shaped cap in the expanded configuration.

The plurality of arms may be at least partially disposed under the sealing element.

The sealing element may comprise a plurality of proximal slots and plurality of distal slots, and wherein the plurality of arms extend out of the plurality of proximal slots and are disposed along an outer surface of the sealing element, and wherein the plurality of arms are inserted into the plurality of distal slots.

The sealing element may comprise a disc or a fabric cover. The sealing element may comprise a filament threaded through tissue adjacent the left atrial appendage, and actuation of the filament closes an ostium of the left atrial appendage.

The sealing element may comprise an expandable balloon, an expandable polymer, or an expandable sponge.

Either the anchor element or the sealing element may be self-expanding. A coating may be disposed over at least a portion of the anchor element or the sealing element, and the coating may be configured to promote endothelialization.

In another aspect, a system for sealing a left atrial appendage comprises the device described above and a delivery catheter, wherein the device is releasably coupled to the delivery catheter. The delivery catheter may comprise an inner shaft and an outer sheath slidably disposed thereover. The delivery catheter may be threadably coupled to the device. The system may include an expandable member such as a balloon.

The delivery catheter may comprise an inner-most shaft threadably coupled with the anchoring element; a deployment guide shaft slidably disposed over the inner-most shaft and engaged with the anchoring element shaft; a proximal seal control shaft disposed over the deployment guide shaft and releasably coupled with the sealing element and the deployment guide shaft; and an outer sheath disposed over the proximal seal control shaft constraining self-expansion of the sealing element. The system may further comprise a guidewire and the delivery catheter may be slidably disposed over the guidewire.

In still another aspect, a method for sealing a left atrial appendage comprises advancing a sealing device to the left atrial appendage, expanding an anchoring element on the sealing device and anchoring the sealing device to tissue in or adjacent the left atrial appendage, and expanding a sealing element on the sealing device and sealing the left atrial appendage thereby preventing or reducing thrombus embolization therefrom.

Advancing the sealing device may comprise advancing a delivery catheter carrying the sealing device transeptally to the left atrium. Expanding the anchoring element may comprise retracting a sheath away from the anchoring element thereby allowing the anchoring element to self-expand. Or the anchoring element may be pushed out of the delivery catheter by the pusher on the proximal end of the catheter. Expanding the sealing element may comprise retracting a sheath away from the sealing element thereby allowing the sealing element to self-expand and close the ostium of the appendage effectively excluding it from the blood circulation circuit. Advancing the sealing element or device may comprise advancing the sealing device over a guidewire. Expanding the sealing element or device may comprise expanding a polymer, a sponge or a balloon.

A coating may be disposed over at least a portion of the anchor element or the sealing element, and the coating may be configured to promote endothelialization. The coating may comprise any suitable polymer, such as biocompatible polymers, including polymers that can be derivatized (e.g., covalently) using methods known in the art, to include a polypeptide on at least a portion of the polymer surface. Examples of suitable polymers include Polyethylene Terephthalate (PET), Poly(tetrafluoroethylene) (PTFE), Poly(vinyl alcohol) (PVA), poly(N-2-hydroxypropyl methacyrlamide), Poly(ethylene) (PE), Poly(propylene) (PP), Poly(methylmethacrylate) (PMM), Ethylene-co-vinylacetate (EVA), Poly(dimethylsiloxane) (PDMS), Poly(ether urethanes) (PU), Poly(sulfones) (PS), Poly(ethyleneoxide), Poly(ethyleneoxide-co-propylene oxide) (PEO-PPO), and the like. The polymer surface can be derivatized to include a polypeptide on at least a portion of the polymer surface before the polymer is located on at least a portion of the anchor element or the sealing element described herein or the polymer surface can be derivatized after the polymer is located on either element (e.g., before use), even if either or both elements have already been located in the device for sealing a left atrial appendage. In other words, the polymer surface located on either element can be derivatized after one or both elements are located in the device and before the device is used.

The polymer surface can be derivatized to include a polypeptide on at least a portion of the polymer surface by using any suitable method known in the art. For example, the polymer can comprise reactive groups, such as hydroxyl (-OH) and amine groups (e.g., -NH₂), that allow for the direct coupling of the polypeptide to the polymer surface via, e.g., the polypeptide terminal carboxylic acid (-CO₂H), using known methods. Such reactive groups can "natively" be a part of the polymer (e.g., EVA and PVA natively contain -OH groups on the polymer backbone); the polymer can be plasma-treated (e.g., ammonia helical resonator plasma (HRP) treatment) to form reactive groups on at least a portion of the polymer surface; or the reactive groups can be part of a linker, as described herein.

Whether the reactive groups are "native" or installed, at least a portion of the polymer surface that comprises groups such as hydroxyl and amine groups, or combinations thereof, can be treated with an activating reagent (e.g., disuccinimidyl glutarate (DSG)) to give an activated polymer surface. The activated polymer surface can then be contacted with a polypeptide to give a polypeptide-derivatized polymer surface. Thus, for example, a polypeptide can be coupled to at least a portion of the polymer surface by using an intermediary linking moiety that serves to link at least a portion of the polymer surface to the polypeptide via the linking moiety. More specifically, at least a portion of a polymer can be contacted with a polyfunctional linking reagent (e.g., difunctional) (e.g., ethylene diamine) to give at least a portion of the polymer surface that has been derivatized with a linker comprising at least one reactive group (e.g., at least one -OH or -NH₂ group). The polymer surface that has been derivatized with a linker comprising at least one reactive group can then be treated with an activating reagent (e.g., disuccinimidyl glutarate (DSG)) to give an activated linker. The activated linker can, in turn, be contacted with the polypeptide to give the following: 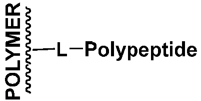 wherein L is an intermediary linking moiety.

In some examples, the polymer is PET. The PET can be hydroxylated. The PET can be aminated. The polymer can be bonded to a polypeptide comprising amino acids GTPGPQGIAGQRGVV, which is generally referred to herein as "polypeptide P15," "P15 polypeptide or simply "P15." The polymer can comprise Expanded Polytetrafluoroethylene (ePTFE). The ePTFE can be bonded to polypeptide P15.

A coating may be disposed over at least a portion of the system. The coating may comprise a polymer. The polymer may comprise PET. The PET may be hydroxylated. The PET is aminated. The polymer may be bonded to a polypeptide comprising amino acids GTPGPQGIAGQRGVV. The polymer may comprise ePTFE. The ePTFE may be bonded to polypeptide P15.

Also described herein is a method for making a coating comprising: hydroxylating a PET and bonding the PET to a polypeptide. The polypeptide may comprise the amino acids GTPGPQGIAGQRGVV. The method may further include the step of coating at least a portion of a surface of a device for sealing a left atrial appendage with the coating. The device may comprise: an anchor element configured to anchor the device to tissue in or adjacent the left atrial appendage; a sealing element configured seal the left atrial appendage and prevent thrombus from embolizing therefrom; and a coupling element joining the anchor element with the sealing element. The method may further include the step of coating at least a portion of a surface of a system for sealing a left atrial appendance with the coating. The system may comprise: the device described herein and a delivery catheter, wherein the device is coupled to the delivery catheter.

Also described herein is a method for making a coating comprising: aminating a PET and bonding the PET to a polypeptide. The polypeptide may comprise the amino acids GTPGPQGIAGQRGVV. The method may further include the step of coating at least a portion of a surface of a device for sealing a left atrial appendage with the coating. The device may comprise: an anchor element configured to anchor the device to tissue in or adjacent the left atrial appendage; a sealing element configured seal the left atrial appendage and prevent thrombus from embolizing therefrom; and a coupling element joining the anchor element with the sealing element. The method may further include the step of coating at least a portion of a surface of a system for sealing a left atrial appendance with the coating. The system may comprise: the device described herein and a delivery catheter, wherein the device is coupled to the delivery catheter.

The above detailed description includes references to the accompanying drawings, which form a part of the detailed description. The drawings show, by way of illustration, specific embodiments in which the invention can be practiced. These embodiments are also referred to herein as "examples." Such examples can include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

In the event of inconsistent usages between this document and any documents, the usage in this document controls.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

The above description is intended to be illustrative, and not restrictive. For example, the above-described examples (or one or more aspects thereof) may be used in combination with each other. Other embodiments can be used, such as by one of ordinary skill in the art upon reviewing the above description. The Abstract is provided to allow the reader to quickly ascertain the nature of the technical disclosure. It is submitted with the understanding that it will not be used to interpret or limit the scope or meaning of the claims. Also, in the above Detailed Description, various features may be grouped together to streamline the disclosure. This should not be interpreted as intending that an unclaimed disclosed feature is essential to any claim. Rather, inventive subject matter may lie in less than all features of a particular disclosed embodiment. Thus, the following claims are hereby incorporated into the Detailed Description as examples or embodiments, with each claim standing on its own as a separate embodiment, and it is contemplated that such embodiments can be combined with each other in various combinations or permutations. The scope of the invention should be determined with reference to the appended claims.

## Claims

1. A device for sealing a left atrial appendage, said device comprising:
an anchor element (1812; 2206; 3224) configured to anchor the device to tissue in or adjacent the left atrial appendage, wherein the anchor element (1812; 2206; 3224) comprises a plurality of arms (1814; 2210; 3208) with barbs (810; 2208) disposed thereon having an expanded configuration for anchoring to the tissue in the left atrial appendage, and a collapsed configuration for delivery to the left atrial appendage, and
wherein the plurality of arms (1814; 2210; 3208) form an arcuate basket in the expanded configuration;
a sealing element (1804; 2202; 3220) configured seal the left atrial appendage and prevent thrombus from embolizing therefrom, wherein the plurality of arms (1814; 2210; 3208) are at least partially disposed under the sealing element (1804; 2202; 3220), and
a coupling element (1808; 2204) joining the anchor element (1812; 2206; 3224) with the sealing element (1804; 2202; 3220);
the device being **characterized in that** the sealing element (1804; 2202; 3220) comprises a plurality of proximal slots (2402; 3204) and plurality of distal slots (2404 or 2406; 3202), and wherein the plurality of arms extend out of the plurality of proximal slots and are disposed along an outer surface of the sealing element (1804; 2202; 3220), and wherein the plurality of arms (1814; 2210; 3208) are inserted into the plurality of distal slots.

2. The device of claim 1, wherein the sealing element (1804) comprises a plurality of arms (1806) having an expanded configuration for sealing the left atrial appendage, and a collapsed configuration for delivery to the left atrial appendage, and
wherein the plurality of arms (1806) form a diamond shaped cap in the expanded configuration.

3. The device of claim 1, wherein the anchor element (1812; 2206) or the sealing element (1804; 2204) are self-expanding.

4. The device of claim 1, wherein the sealing element (1804; 2202) comprises a disc.

5. The device of claim 1, further comprising a coating disposed over at least a portion of the anchor element or the sealing element, wherein the coating is configured to promote endothelialization.

6. The device of claim 1, wherein the sealing element comprises a fabric or polymer cover.

7. A system for sealing a left atrial appendage, said system comprising:
the device (1800; 2200) of claim 1; and
a delivery catheter, wherein the device (1800; 2200) is releasably coupled to the delivery catheter.

8. The system of claim 7, wherein the delivery catheter comprises:
an inner-most shaft threadably coupled with the anchoring element;
a deployment guide shaft (3228) slidably disposed over the inner-most shaft and engaged with the anchoring element;
a proximal seal control shaft (3216) disposed over the deployment guide shaft (3228) and releasably coupled with the sealing element and the deployment guide shaft (3228); and
an outer sheath disposed over the proximal seal control shaft constraining self-expansion of the sealing element.

9. The system of claim 7, wherein the delivery catheter is threadably coupled the device.

10. The system of claim 7, wherein the delivery catheter comprises an expandable member adjacent a distal end thereof.

11. The device of claim 5, wherein the coating comprises a polymer, and
wherein the polymer comprises PET, or a hydroxylated or animated PET, or
wherein the polymer is bonded to a peptide comprising amino acids GTPGPQGIAGQRGVV, or wherein the polymer comprises ePTFE, or ePTFE bonded to polypeptide P15.

12. A system for sealing a left atrial appendage, said system comprising:
the device of claim 1; and
a delivery catheter, wherein the device is coupled to the delivery catheter.

## Patentansprüche

1. Eine Vorrichtung zum Abdichten eines linken Herzohrs, wobei die genannte Vorrichtung Folgendes umfasst:
ein Ankerelement (1812; 2206; 3224), das zum Verankern der Vorrichtung an Gewebe in dem oder neben dem linken Herzohr ausgelegt ist, wobei das Ankerelement (1812; 2206; 3224) eine Vielzahl von Armen (1814; 2210; 3208) mit darauf angeordneten Widerhaken (810; 2208) umfasst, die eine expandierte Konfiguration zum Verankern an dem Gewebe in dem linken Herzohr und eine zusammengefaltete Konfiguration zum Zuführen zu dem linken Herzohr aufweisen, und
wobei die Vielzahl von Armen (1814; 2210; 3208) in der expandierten Konfiguration einen gekrümmten Korb bilden;
ein Abdichtungselement (1804; 2202; 3220), das dazu ausgelegt ist, das linke Herzohr abzudichten und zu verhindern, dass sich ein Thrombus davon ablöst, wobei die Vielzahl von Armen (1814; 2210; 3208) mindestens teilweise unter dem Abdichtungselement (1804; 2202; 3220) angeordnet sind, und
ein Kopplungselement (1808; 2204), das das Ankerelement (1812; 2206; 3224) mit dem Abdichtungselement (1804; 2202; 3220) verbindet;
wobei die Vorrichtung **dadurch gekennzeichnet ist, dass** das Abdichtungselement (1804; 2202; 3220) eine Vielzahl von proximalen Schlitzen (2402; 3204) und eine Vielzahl von distalen Schlitzen (2404 oder 2406; 3202) umfasst, und wobei sich die Vielzahl von Armen aus der Vielzahl von proximalen Schlitzen erstrecken und entlang einer Außenfläche des Abdichtungselements (1804; 2202;3220) angeordnet sind, und wobei die Vielzahl von Armen (1814; 2210; 3208) in die Vielzahl von distalen Schlitzen eingesteckt sind.

2. Vorrichtung nach Anspruch 1, wobei das Abdichtungselement (1804) eine Vielzahl von Armen (1806) umfasst, die eine expandierte Konfiguration zum Abdichten des linken Herzohrs und eine zusammengefaltete Konfiguration zum Zuführen zu dem linken Herzohr aufweisen, und
wobei die Vielzahl von Armen (1806) in der expandierten Konfiguration eine rautenförmige Kappe bilden.

3. Vorrichtung nach Anspruch 1, wobei das Ankerelement (1812; 2206) oder das Abdichtungselement (1804; 2204) selbstexpandierend ist.

4. Vorrichtung nach Anspruch 1, wobei das Abdichtungselement (1804; 2202) eine Scheibe umfasst.

5. Vorrichtung nach Anspruch 1, die ferner eine Beschichtung umfasst, die über mindestens einem Anteil des Ankerelements oder des Abdichtungselements angeordnet ist, wobei die Beschichtung dazu ausgelegt ist, eine Endothelialisierung zu fördern.

6. Vorrichtung nach Anspruch 1, wobei das Abdichtungselement eine Textil- oder Polymerabdeckung umfasst.

7. Ein System zum Abdichten eines linken Herzohrs, wobei das genannte System Folgendes umfasst:
die Vorrichtung (1800; 2200) nach Anspruch 1; und
einen Zuführkatheter, wobei die Vorrichtung (1800; 2200) lösbar mit dem Zuführkatheter gekoppelt ist.

8. System nach Anspruch 7, wobei der Zuführkatheter Folgendes umfasst:
einen innersten Schaft, der verschraubbar mit dem Verankerungselement gekoppelt ist;
einen Freigabeführungsschaft (3228), der gleitfähig über dem innersten Schaft angeordnet ist und in Eingriff mit dem Verankerungselement steht;
einen proximalen Abdichtungskontrollschaft (3216), der über dem Freigabeführungsschaft (3228) angeordnet ist und lösbar mit dem Abdichtungselement und dem Freigabeführungsschaft (3228) gekoppelt ist; und
eine äußere Schleuse, die über dem proximalen Abdichtungskontrollschaft angeordnet ist, die die Selbstexpansion des Abdichtungselements einschränkt.

9. System nach Anspruch 7, wobei der Zuführkatheter verschraubbar mit der Vorrichtung gekoppelt ist.

10. System nach Anspruch 7, wobei der Zuführkatheter ein expandierbares Element neben einem distalen Ende davon umfasst.

11. Vorrichtung nach Anspruch 5, wobei die Beschichtung ein Polymer umfasst und
wobei das Polymer PET oder ein hydroxyliertes oder aminiertes PET umfasst, oder
wobei das Polymer an ein Peptid gebunden ist, das die Aminosäuren GTPGPQGIAGQRGVV umfasst oder wobei das Polymer ePTFE oder an Polypeptid P15 gebundenes ePTFE umfasst.

12. Ein System zum Abdichten eines linken Herzohrs, wobei das genannte System Folgendes umfasst:
Vorrichtung nach Anspruch 1; und
einen Zuführkatheter, wobei die Vorrichtung mit dem Zuführkatheter gekoppelt ist.

## Revendications

1. Dispositif destiné à la fermeture étanche d'un appendice auriculaire gauche, comprenant :
un élément d'ancre (1812 ; 2206 ; 3224) configuré pour ancrer le dispositif à un tissu dans ou adjacent à l'appendice auriculaire gauche, dans lequel l'élément d'ancre (1812 ; 2206 ; 3224) comprend une pluralité de bras (1814 ; 2210 ; 3208) avec des barbes (810 ; 2208) disposées sur ceux-ci ayant une configuration déployée pour l'ancrage au tissu dans l'appendice auriculaire gauche, et une configuration repliée pour la délivrance à l'appendice auriculaire gauche, et
dans lequel la pluralité de bras (1814 ; 2210 ; 3208) forment un panier arqué dans la configuration déployée ;
un élément d'étanchéité (1804 ; 2202 ; 3220) configuré pour fermer de manière étanche l'appendice auriculaire gauche et empêcher un thrombus de s'emboliser à partir de celui-ci, dans lequel la pluralité de bras (1814 ; 2210 ; 3208) sont au moins partiellement disposés sous l'élément d'étanchéité (1804 ; 2202 ; 3220), et
un élément de couplage (1808 ; 2204) reliant l'élément d'ancre (1812 ; 2206 ; 3224) à l'élément d'étanchéité (1804 ; 2202 ; 3220) ;
le dispositif étant **caractérisé en ce que** l'élément d'étanchéité (1804 ; 2202 ; 3220) comprend une pluralité de fentes proximales (2402 ;3204) et une pluralité de fentes distales (2404 ou 2406 ; 3202), et dans lequel la pluralité de bras s'étendent hors de la pluralité de fentes proximales et sont disposés le long d'une surface externe de l'élément d'étanchéité (1804 ; 2202 ; 3220), et dans lequel la pluralité de bras (1814 ; 2210 ; 3208) sont insérés dans la pluralité de fentes distales.

2. Dispositif selon la revendication 1, dans lequel l'élément d'étanchéité (1804) comprend une pluralité de bras (1806) ayant une configuration déployée pour la fermeture étanche de l'appendice auriculaire gauche, et une configuration repliée pour la délivrance à l'appendice auriculaire gauche, et
dans lequel la pluralité de bras (1806) forment un capuchon en forme de diamant dans la configuration déployée ;

3. Dispositif selon la revendication 1, dans lequel l'élément d'ancre (1812 ; 2206) ou l'élément d'étanchéité (1804 ; 2204) sont auto-déployables.

4. Dispositif selon la revendication 1, dans lequel l'élément d'étanchéité (1804 ; 2202) comprend un disque.

5. Dispositif selon la revendication 1, comprenant en outre un revêtement disposé sur au moins une partie de l'élément d'ancre ou de l'élément d'étanchéité, dans lequel le revêtement est configuré pour favoriser l'endothélialisation.

6. Dispositif selon la revendication 1, dans lequel l'élément d'étanchéité comprend un revêtement en tissu ou en polymère.

7. Système destiné à la fermeture étanche d'un appendice auriculaire gauche, ledit système comprenant :
le dispositif (1800 ; 2200) selon la revendication 1 ; et
un cathéter de délivrance, dans lequel le dispositif (1800 ; 2200) est couplé de manière libérable au cathéter de délivrance.

8. Système selon la revendication 7, dans lequel le cathéter de délivrance comprend :
un arbre le plus interne couplé par filetage à l'élément d'ancrage ;
un arbre de guide de déploiement (3228) disposé de manière coulissante sur l'arbre le plus interne et entré en prise avec l'élément d'ancrage ;
un arbre de commande de joint étanche proximal (3216) disposé sur l'arbre de guide de déploiement (3228) et couplé de manière libérable à l'élément d'étanchéité et à l'arbre de guide de déploiement (3228) ; et
une gaine extérieure disposée sur l'arbre de commande de joint étanche proximal contraignant l'auto-déploiement de l'élément d'étanchéité.

9. Système selon la revendication 7, dans lequel le cathéter de délivrance est couplé par filetage au dispositif.

10. Système selon la revendication 7, dans lequel le cathéter de délivrance comprend un élément capable de déploiement adjacent à une extrémité distale de celui-ci.

11. Dispositif selon la revendication 5, dans lequel le revêtement comprend un polymère, et
dans lequel le polymère comprend du PET, ou un PET hydroxylé ou aminé, ou
dans lequel le polymère est lié à un peptide comprenant les acides aminés GTPGPQGIAGQRGVV, ou dans lequel le polymère comprend de l'ePTFE, ou de l'ePTFE lié au polypeptide P15.

12. Système destiné à la fermeture étanche d'un appendice auriculaire gauche, ledit système comprenant :
le dispositif selon la revendication 1 ; et
un cathéter de délivrance, dans lequel le dispositif est couplé au cathéter de délivrance.
